# EUROPÄISCHE PATENTSCHRIFT

(11) **EP 1 841 731 B1**
(45) Veröffentlichungstag und Bekanntmachung des Hinweises auf die Patenterteilung: **22.08.2012**
(21) Anmeldenummer: 06700901.9
(22) Anmeldetag: 16.01.2006
(51) Int. Cl.: C07C 311/60, C07D 211/16, C07D 295/096, C07D 295/32, A61K 31/18, A61K 31/495, A61K 31/4453, A61K 31/5375, A61P 3/10, A61P 5/50

(54) **SUBSTITUIERTE N-AMINOMETHYLENSULFONAMIDE, IHRE HERSTELLUNG UND VERWENDUNG ALS ARZNEIMITTEL**
SUBSTITUTED AMINOMETHYLENE SULPHONAMIDES, PRODUCTION AND USE THEREOF AS MEDICAMENTS
N-AMINOMETHYLENSULFONAMIDE SUBSTITUE, SA PRODUCTION ET SON UTILISATION COMME MEDICAMENT

(30) Priorität: 17.01.2005 DE 102005002130
(43) Veröffentlichungstag der Anmeldung: 10.10.2007
(73) Patentinhaber: SANOFI, 75008 Paris (FR)
(72) Erfinder: SCHOENAFINGER, Karl, 63755 Alzenau (DE); PETRY, Stefan, 65926 Frankfurt am Main (DE); MUELLER, Guenter, 65843 Sulzbach (DE); BARINGHAUS, Karl-Heinz, 61200 Wölfersheim (DE)
(74) Vertreter: Then, Johann
(86) Internationale Anmeldenummer: PCT/EP2006/000313
(87) Internationale Veröffentlichungsnummer: WO 2006/074957

(56) Entgegenhaltungen:
- WO-A-03/051841
- DATABASE CAPLUS [Online] CHEMICAL ABSTRACTS SERVICE, COLUMBUS, OHIO, US; SHANI, J. ET AL: "Structure activity correlation for diuretic furosemide congeners" XP002379192 gefunden im STN Database accession no. 1983:65112 & PHARMACOLOGY , 26(3), 172-80 CODEN: PHMGBN; ISSN: 0031-7012, 1983,

## Beschreibung

Die Erfindung betrifft Verbindungen der allgemeinen Formel (I), wobei die Definitionen der Substituenten R¹ bis R⁵, A und X im nachfolgenden Text aufgeführt sind, sowie deren physiologisch verträglichen Salze, Verfahren zur Herstellung dieser Verbindungen und deren Verwendung als Arzneimittel.

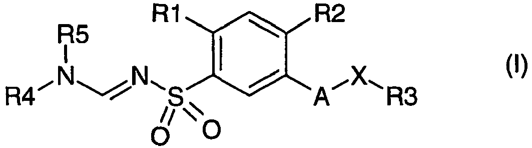

Diese Verbindungen sind Inhibitoren der hormonsensitiven Lipase (HSL).

Aus der Literatur ist es bereits bekannt, dass Verbindungen, die sich als Inhibitoren von HSL eignen, zur Behandlung von Diabetes Mellitus eingesetzt werden können. So werden hierfür in WO 2004/035550 Benzotriazol-Derivate eingesetzt. In WO 03/105860 werden Derivate von Borsäure und Borsäureestern, in WO 03/051842 werden u.a. Amide, die eine hydrolysierbare Gruppe aufweisen, als HSL-Inhibitoren verwendet. Dass sich als HSL-Inhibitoren auch Aminomethylensulfonamide eignen, ist bis jetzt noch nicht beschrieben worden.

Zwar sind in der Literatur bereits Sulfonamid-Verbindungen bekannt, die sich jedoch von denjenigen der vorliegenden Erfindung durch ein unterschiedliches Substitutionsmuster sowie andere Verwendungen (Indikationen) unterscheiden.

In R. Neidlein et al., Monatshefte für Chemie 116 (1985), Seiten 651 bis 660 werden Synthesen und spektroskopische Eigenschaften von Alkylmercaptoalkylaminomethylensulfonamiden beschrieben. Diese Verbindungen weisen am Benzol-Fragment zwingend zwei Aminomethylensulfonamid-Substituanten in Meta-Position auf, während die erfindungsgemäßen Verbindungen nur einen solchen Substituenten haben. Die in R. Neidlein et al. beschriebenen Verbindungen werden nicht mit einer etwaigen Verwendung als Arzneimittel in Verbindung gebracht.

In US 3,009,910 werden 2,4-Disulfamyl-anillin-Derivate sowie deren Verwendung als Arzneimittel mit diuretischer Wirkung beschrieben. Während bei den erfindungsgemäßen Verbindungen der Methylensulfonamid-Substituent in ortho-Position zu R¹ (beispielsweise Halogen) und in para-Position zu R² (beispielsweise -NH₂) steht, ist bei den Verbindungen gemäß US 3,009,910 dieses Substitutionsmuster genau umgekehrt. Der Methylensulfonamid-Substituent steht am zentralen Benzol-Fragment in ortho-Position zu einem Amino-Substituenten und in para-Position zu einem Halogen-Substitüenten. Ein Zusammenhang der in US 3,009,910 beschriebenen Verbindungen mit der Behandlung von Diabetes beziehungsweise die Verwendung dieser Verbindungen als Inhibitor von HSL ist in dieser Schrift jedoch nicht offenbart.

EP-A 0 008 433 betrifft Sulfamoylbenzol-Derivate sowie deren Verwendung als Diuretika und Saluretika. Die darin beschriebenen Verbindungen unterscheiden sich von den erfindungsgemäßen Sulfonamiden dahingehend, dass das zentrale Benzol-Fragment in ortho-Position zu einem Halogen-Substituenten einen unsubstituierten Sulfamoyl-Substituenten aufweist. Zur Synthese der in EP-A 0 008 433 beschriebenen Verbindungen werden mehrere Wege aufgezeigt, wobei unter anderem die folgenden Ausgangsmaterialien der Formeln A und B eingesetzt-werden;

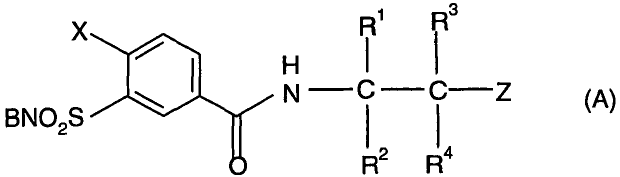

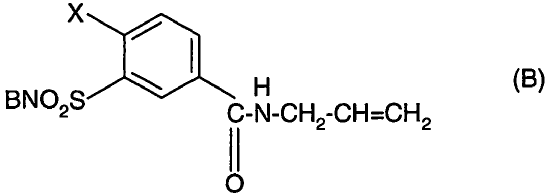

In den Verbindungen der Formel A sind die Reste R¹ bis R⁴ unabhängig voneinander als Wasserstoff oder ein Alkylrest mit 1 bis 4 C-Atomen, wobei einer der Reste R¹ bis R⁴ auch eine Carboxy-, Hydroxymethyl- oder eine Alkyloxycarbonyl-Gruppe mit höchstens 5 C-Atomen oder einer oder zwei der Reste R¹ bis R⁴ iso-Propyl, iso-Butyl, tert.-Butyl, Phenyl oder Cycloalkyl mit 5 bis 6 C-Atomen bedeuten können, X als Halogen, Z als Abgangsgruppe (Halogen, Hydroxy, tri-Alkylammonium, Mesylat oder Tosylat) und B entweder als 2 Wasserstoffatome oder eine Schutzgruppe der Formel: =CR⁵-NR⁶R⁷ definiert, wobei die Reste R⁵ bis R⁷ unabhängig voneinander eine Alkylgruppe mit 1 bis 4 C-Atomen und R⁵ gegebenenfalls auch Wasserstoff sein können. Die Verbindungen der Formeln A und B können jeweils auch zu Zwischenverbindungen umgelagert werden, bei denen der zum Halogen-Substituenten sich in para-Position befindliche Substituent als - C(O)-O-CR³R⁴-CR¹R²-NH₂ definiert ist. Die vorgenannten Zwischenverbindungen, die in EP-A 0 008 433 als solche offenbart sind und für die keine Verwendung als Arzneimittel beschrieben ist, sind nicht Gegenstand der vorliegenden Erfindung.

DE-A 25 18 999 betrifft weitere Sulfamylbenzoesäure-Derivate, die ebenfalls als Diuretica oder Saluretica verwendet werden können. Entsprechend zu EP-A 0 008 433 unterscheiden sich auch die in DE-A 25 18 999 beschriebenen pharmazeutisch aktiven Verbindungen der allgemeinen Formel I durch das Substitutionsmuster am zentralen Benzol-Fragment, das in ortho-Position zu einem Halogen-Substituenten einen Amino-Substituenten aufweist, während die erfindungsgemäßen Verbindungen an dieser Position unsubstituiert sind. Zur Herstellung der in DE-A 25 18 999 beschriebenen Verbindungen geht man von Sulfamylbenzoesäure-Derivaten einer allgemeinen Formel m aus, die am zentralen Benzol-Fragment in para-Postition zum Halogen-Sustituenten Y einen (-COOR)-Substituenten aufweisen, wobei R für Wasserstoff, einen Alkyl- oder Cykloalkylrest mit bis zu 6 Kohlenstoffatomen steht. Diese vorgenannten Zwischenverbindungen der allgemeinen Formel III von DE-A 25 18 999, die darin als solche offenbart sind und für die keine Verwendung als Arzneimittel beschrieben ist, sind nicht Gegenstand der vorliegenden Erfindung.

In EP-A 0 324 988 sowie EP-A 0 324 184 werden weitere diuretisch und salzentziehend wirkende Arzneimittelpräparate (von jeweils) einer allgemeinen Formel I beschrieben, die in ortho-Position zum Chlor-Substituenten des zentralen Benzol-Fragmentes einen unsubstituierten Sulfamyl-Substituenten aufweisen. Zusätzlich weisen die Verbindungen der Formel I in EP-A 0 324 988 in para-Position zum Chlor-Substituenten einen AmidSubstituenten auf, der wiederum mit unsubstituiertem oder zumindest monosubstituiertem Benzimidazolyl substituiert ist, in EP-A 0 324 184 ist der Amid-Substituent mit unsubstituiertem oder zumindest monosubstituiertem (1,2,3,4-Tetrahydroisochinolinyl) substituiert. Sinngemäß zu EP-A 0 008 433 werden die in den beiden vorgenannten EP-Anmeldungen genannten, pharmazeutischen Wirkstoffe aus Edukten synthetisiert, die anstelle eines unsubstituierten Sulfamyl-Substituenten einen Dialkylaminomethylen-Sulfamyl-Substituenten aufweisen. Die in EP-A 0 324 988 bzw. EP-A 0 324 184 beschriebenen Zwischenverbindungen, die als solche offenbart sind und für die keine Verwendung als Arzneimittel beschrieben ist, sind nicht Gegenstand der vorliegenden Erfindung.

Da Krankheiten wie Diabetes Mellitus, die durch die Inhibierung von HSL behandelt werden können, eine ernstzunehmenden Gefahr für die Gesundheit von Menschen und anderen Säugern darstellen, besteht ein großer Bedarf nach neuen Arzneimitteln, die über ein vorteilhaftes therapeutisches Profil zur Behandlung von solchen Krankheiten verfügen. Der vorliegenden Anmeldung liegt deshalb die Aufgabe zugrunde, neue Verbindungen bereitzustellen, die einen inhibitorischen Effekt auf HSL haben.

Die Aufgabe wird durch substituierte N-Aminomethylensulfonamide der allgemeinen Formel (I) gelöst. 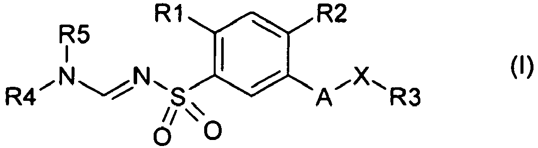 worin bedeuten:
- R1: ist Wasserstoff, Halogen oder -CF₃;
- R2: ist Wasserstoff, -NH₂, -NH(C₁-C₃-Alkyl), -N(C₁-C₃-Alkyl)₂ oder C₁-C₃-Alkoxy und R1 und R2 können nicht gleichzeitig Wasserstoff sein, wobei Alkyl oder Alkoxy unsubstituiert ist;
- R3: ist unsubstituiertes oder zumindest monosubstituiertes C₁-C₁₀-Alkyl, C₂-C₁₀-Alkenyl, Aryl, Heterocyclyl oder Heteroaryl, wobei die Substituenten ausgewählt sind aus der Gruppe bestehend aus: Aryl, Heteroaryl, Heterocyclyl, Aryl-(C₁-C₆-alkyl)-, Heterocyclyl-(C₁-C₆-alkyl)-, Heteroaryl-(C₁-C₆-alkyl)-, -O-Aryl, Fluor, Chlor, Brom, -CF₃, - OCF₃, -NO₂, -CN, -C(O)R7, Hydroxy, C₁-C₆-Alkyl und C₁-C₃-Alkoxy, und die Aryl-, Heteroaryl- und Heterocyclyl-Fragmente dieser Substituenten können wiederum mit Fluor, Chlor, Brom, Oxo, -CF₃, -OCF₃, -NO₂, -CN, Hydroxy, C₁-C₃-Alkyl oder C₁-C₃-Alkoxy zumindest monosubstituiert sein;
- R4 und R5: sind unabhängig voneinander Wasserstoff, unsubstituiertes oder zumindest monosubstituiertes C₁-C₁₀-Alkyl oder Aryl-(C₁-C₆-alkyl)-, wobei die Substituenten ausgewählt sind aus der Gruppe bestehend aus: Aryl, Heteroaryl, Heterocyclyl, Aryl-(C₁-C₆-alkyl)-, Heterocyclyl-(C₁-C₆-alkyl)-, Heteroaryl-(C₁-C₆-alkyl)-, -O-Aryl, Fluor, Chlor, Brom, -CF₃, - OCF₃, -NO₂, -CN, -C(O)R7, Hydroxy, C₁-C₆-Alkyl und C₁-C₃-Alkoxy, und die Aryl-, Heteroaryl- und Heterocyclyl-Fragmente dieser Substituenten können wiederum mit Fluor, Chlor, Brom, Oxo, -CF₃, -OCF₃, -NO₂, -CN, Hydroxy, C₁-C₃-Alkyl oder C₁-C₃-Alkoxy zumindest monosubstituiert sein; oder
- R4 und R5: bilden zusammen mit dem Stickstoffatom, an das sie gebunden sind, unsubstituiertes oder zumindest monosubstituiertes Heterocyclyl, wobei die Substituenten ausgewählt sind aus: Halogen, C₁-C₆-Alkyl, C₁-C₆-Alkoxy, Phenyl, Oxo, -NH₂, -NH(C₁-C₃-Alkyl), -N(C₁-C₃-Alkyl)₂, -OCF₃, -CF₃ oder Hydroxy;
- R6: ist Wasserstoff, -SO₂(C₁-C₃-Alkyl), unsubstituiertes oder zumindest monosubstituiertes C₁-C₁₀-Alkyl, C₂-C₁₀-Alkenyl oder Aryl-(C₁-C₆-alkyl)-,
wobei die Substituenten ausgewählt sind aus: Halogen, C₁-C₆-Alkyl, C₁-C₆-Alkoxy, Phenyl, Oxo, -NH₂, -NH(C₁-C₃-Alkyl), -N(C₁-C₃-Alkyl)₂, -OCF₃, -CF₃ oder Hydroxy; oder
- R6: bildet zusammen mit R3 und X, sofern X gleich N ist, unsubstituiertes oder zumindest monosubstituiertes Heterocyclyl, wobei die Substituenten ausgewählt sind aus der Gruppe bestehend aus: Aryl, Heteroaryl, Heterocyclyl, Aryl-(C₁-C₆-alkyl)-, Heterocyclyl-(C₁-C₆-alkyl)-, Heteroaryl-(C₁-C₆-alkyl)-, -O-Aryl, Fluor, Chlor, Brom, -CF₃, - OCF₃, -NO₂, -CN, -C(O)R7, Hydroxy, C₁-C₆-Alkyl und C₁-C₃-Alkoxy, und die Aryl-, Heteroaryl- und Heterocyclyl-Fragmente dieser Substituenten können wiederum mit Fluor, Chlor, Brom, Oxo, -CF₃, -OCF₃, -NO₂, -CN, Hydroxy, C₁-C₃-Alkyl oder C₁-C₃-Alkoxy zumindest monosubstituiert sein;
- R7: ist C₁-C₃-Alkoxy, -O-Phenyl, C₁-C₃-Alkyl, -NH₂, -NH(C₁-C₃-Alkyl), -N(C₁-C₃-Alkyl)₂ oder Phenyl, und die Phenyl-Fragmente von R7 können wiederum mit Fluor, Chlor, Brom, -CF₃, -OCF₃, -NO₂, -CN, Hydroxy, C₁-C₃-Alkyl oder C₁-C₃-Alkoxy zumindest monosubstituiert sein;
- A: ist SO₂, CO oder CH₂;
- X: ist NR6 oder O;
Heteroaryl ist ein 5 bis 10-gliedriger, aromatischer, mono- oder bicyclischer Heterocyclus, der ein oder mehrere Heteroatome ausgewählt aus N, O und S enthält;
Aryl ist ein 5 bis 10-gliedriger, aromatischer, Mono- oder Bicyclus;
Heterocyclyl ist ein 5 bis 10-gliedriger, nicht-aromatischer, mono- oder bicyclischer Heterocyclus, der ein oder mehrere Heteroatome ausgewählt aus N, O und S enthält;
oder ein physiologisch verträgliches Salz davon;
unter der Voraussetzung, dass wenn R2 Wasserstoff und R1 Halogen sind, -A-X-R3 nicht i) -C(O)-O-CH₂-CH₂-NH₂, ii) -C(O)-NH-CH₂-CH=CH₂, iii) -C(O)-NH-CH₂-CH₂-Z, mit Z gleich Halogen, Hydroxy, tri-Alkylammonium, Mesylat oder Tosylat, iv) -C(O)-O-(C₁-C₆-Alkyl), v) -C(O)-NH-Benzimidazolyl oder vi) -C(O)-NH-(1,2,3,4-Tetrahydroisochinolinyl) sind, wobei in i) und iii) das -CH₂-CH₂-Fragment 1 bis 4 Alkylreste R¹ bis R⁴ mit jeweils 1 bis 4 C-Atomen aufweisen kann, wobei einer der Reste R¹ bis R⁴ auch eine Carboxy-, Hydroxymethyl- oder eine Alkyloxycarbonyl-Gruppe mit höchstens 5 C-Atomen oder einer oder zwei der Reste R¹ bis R⁴ iso-Propyl, iso-Butyl, tert.-Butyl, Phenyl oder Cycloalkyl mit 5 bis 6 C-Atomen bedeuten können, und wobei in v) und vi) das Benzimidazolyl- und das (1,2,3,4-Tetrahydroisochinolinyl)-Fragment unsubstituiert oder zumindest monosubstituiert sein können.

Sofern in den Verbindungen gemäß der Formel (I) Gruppen, Fragmente, Reste oder Substituenten wie beispielsweise Aryl, Heteroaryl, Alkyl, Alkoxy usw. mehr als einfach vorhanden sind, haben sie alle unabhängig voneinander die oben aufgeführten Bedeutungen und können somit in jedem (Einzel)-Fall entweder eine identische oder eine voneinander unabhängige Bedeutung haben. Die nachfolgenden Ausführungen gelten-für (beispielsweise) Aryl sowie jeden beliebigen anderen Rest unabhängig von dessen Bezeichnung als Arylgruppe, -substituent, -fragment oder -rest. Ein weiteres Beispiel ist die -N(C₁-C₃-alkyl)₂ Gruppe, in der die beiden Alkylsubstituenten entweder identisch oder unterschiedlich sein können(beispielsweise zweimal Ethyl oder einmal Propyl und einmal Methyl).

Sofern in den vorstehenden Definitionen für Verbindungen gemäß Formel (I) ein Substituent, beispielsweise Aryl, unsubstituiert oder zumindest monosubstituiert mit einer Gruppe von weiteren Substituenten, beispielsweise C₁-C₆-Alkyl, C₁-C₆-Alkoxy, Halogen usw., sein kann, so gilt für diejenigen Fälle, in denen eine Mehrfachsubstitution von Aryl vorliegt, dass die Auswahl aus der Reihe von weiteren Substituenten unabhängig voneinander erfolgt. Somit sind beispielsweise bei einer Zweifachsubstitution von Aryl sämtliche Kombinationen der weiteren Substituenten mit umfasst. Aryl kann somit beispielsweise zweifach-substituiert mit Ethyl sein, Aryl kann jeweils monosubstituiert mit Methyl und Ethoxy sein, Aryl kann jeweils monosubstituiert mit Ethyl und Fluor sein, Aryl kann zweifach-substituiert mit Methoxy sein, usw..

Alkylreste können entweder linear oder verzweigt, acyclisch oder cyclisch sein. Dies trifft auch zu, sofern sie Teil einer anderen Gruppe wie beispielsweise Alkoxygruppen (C₁-C₁₀-Alkyl-O-), Alkoxycarbonylgruppen oder Aminogruppen sind oder wenn sie substituiert sind.

Beispiele für Alkylgruppen sind: Methyl, Ethyl, Propyl, Butyl, Pentyl, Hexyl, Heptyl, Octyl, Nonyl oder Decyl. Dabei sind sowohl die n-Isomere dieser Reste als auch Isopropyl, Isobutyl, Isopentyl, sec-Butyl, tert-Butyl, Neopentyl, 3,3-Dimethylbutyl usw. mit umfasst. Sofern nicht anders beschrieben, beinhaltet der Begriff Alkyl darüber hinaus auch Alkylreste, die unsubstituiert oder gegebenenfalls mit einem oder mehreren weiteren Resten substituiert sind, beispielsweise 1, 2, 3 oder 4 identische oder unterschiedliche Reste, wie beispielsweise Aryl, Heteroaryl, Alkoxy oder Halogen. Dabei können die zusätzlichen Substituenten in jeder beliebigen Position des Alkylrestes auftreten. Weiterhin umfasst der Begriff Alkyl auch Cycloalkyl sowie Cycloalkyl-alkyl- (Alkyl, das wiederum mit Cycloalkyl substituiert ist), wobei Cycloalkyl mindestens 3 Kohlenstoffatome aufweist. Cycloalkyl kann wiederum mit einem oder mehreren Alkylresten substituiert sein. Beispiele für Cycloalkylreste sind: Cyclopropyl, Cyclobutyl, Cyclopentyl, Cyclohexyl, Cycloheptyl, Cyclooctyl, Cyclononyl und Cyclodecyl. Gegebenenfalls kann es sich auch um polycyclische Ringsysteme handeln, wie Decalinyl, Norbornanyl, Bornanyl oder Adamantanyl. Die Cycloalkylreste können unsubstituiert oder gegebenenfalls mit einem oder mehreren weiteren Resten substituiert sein, wie vorstehend bei den Alkylresten beispielhaft aufgeführt.

Beispiele für Alkenyl- und Alkinylgruppen sind: Vinyl, 1-Propenyl, 2-Propenyl (Allyl), 2-Butenyl, 2-Methyl-2-propenyl, 3-Methyl-2-butenyl, Ethinyl, 2-Propinyl (Propargyl), 2-Butinyl oder 3-Butinyl. Der Begriff Alkenyl umfasst hier ausdrücklich auch Cycloalkenylreste sowie Cycloalkenyl-Alkylreste (Alkyl, das mit Cycloalkenyl substituiert ist), die mindestens drei Kohlenstoffatome enthalten. Beispiele für Cycloalkenyl sind: Cyclopentenyl, Cyclohexenyl, Cycloheptenyl und Cyclooctenyl.

Die Alkenylreste können ein bis drei konjugierte oder nicht konjugierte Doppelbindungen (also auch Alk-dienyl- sowie Alk-trienylreste), vorzugsweise eine Doppelbindung in einer geraden oder verzweigten Kette aufweisen, für Alkinylreste gilt das gleiche für die Dreifachbindungen. Die Alkenyl- und Alkinylreste können unsubstituiert oder gegebenenfalls mit einem oder mehreren weiteren Resten substituiert sein, wie vorstehend bei den Alkylresten beispielhaft aufgeführt.

Sofern nicht anders ausgeführt, können die vorgenannten Aryl-, Heteroaryl- und Heterocyclylreste sowohl unsubstituiert sein als auch einen oder mehrere, beispielsweise 1, 2, 3 oder 4 weitere, der vorgenannten Substituenten in jeder beliebigen Position aufweisen. Beispielsweise kann in monosubstituierten Phenylresten der Substituent in der Position 2, 3 oder 4, in disubstituierten Phenylresten können die Substituenten in der 2,3-Position, 2,4-Position, 2,5-Position, 2,6-Position, 3,4-Position oder 3,5-Position sein. In dreifach substituierten Phenylresten können die Substituenten in der 2,3,4-Position, 2,3,5-Position, 2,3,6-Position, 2,4,5-Position, 2,4,6-Position oder 3,4,5-Position sein. In vierfach substituierten Phenylresten können die Substituenten in der 2,3,4,5-Position, der 2,3,4,6-Position oder in der 2,3,5,6-Position sein.

Die vorgenannten beziehungsweise die nachfolgenden Definitionen, die sich auf einwertige Reste beziehen, gelten genauso für zweiwertige Reste wie Phenylen, Naphthylen oder Heteroarylen. Diese zweiwertigen Reste (Fragmente) können mit dem benachbarten Gruppen für jedes beliebige Ring-Kohlenstoffatom verknüpft sein. Im Falle von Phenylenresten kann dies in der 1,2-Position (ortho-Phenylen), 1,3-Position (meta-Phenylen) oder 1,4-Position (para-Phenylen) sein. Im Falle eines 5-gliedrigen Aromaten, der ein Heteroatom enthält, wie beispielsweise Thiophen oder Furan können die beiden freien Bindungen in der 2,3-Position, 2,4-Position, 2,5-Position oder der 3,4-Position sein. Ein zweiwertiger Rest, der von einem 6-gliedrigen Aromaten mit einem Heteroatom abgeleitet ist, wie beispielsweise Pyridin, kann ein 2,3-, 2,4-, 2,5-, 2,6-, 3,4- oder 3,5-Pyridindiylrest sein. Im Falle von unsymmetrischen zweiwertigen Resten beinhaltet die vorliegende Erfindung auch alle Positionsisomere, d.h. im Falle von beispielsweise einem 2,3-Pyridindiylrest ist die Verbindung, in der die eine benachbarte Gruppe sich in der Position 2 und die andere benachbarte Gruppe in der Position 3 sich befindet, genauso wie die Verbindung, in der die eine benachbarte Gruppe in der Position 3 und die andere benachbarte Gruppe sich in der Position 2 befindet, mit umfasst.

Soweit nicht anders aufgeführt, sind Heteroarylreste, Heteroarylenreste, Hetetrocyclylreste und Heterocyclylenreste sowie Ringe, die durch zwei an Stickstoff gebundene Gruppen gebildet werden, vorzugsweise abgeleitet von vollständig gesättigten, teilweise oder ganz ungesättigten Heterocyclen (d.h. Heterocycloalkane, Heterocycloalkene, Heteroaromaten), die 1, 2, 3 oder 4 Heteroatome enthalten, die sowohl unterschiedlich als auch gleich sein können. Vorzugsweise sind sie von Heterocyclen abgeleitet, die 1, 2 oder 3, besonders bevorzugt 1 oder 2, Heteroatome enthalten, die gleich oder unterschiedlich sein können. So lange nicht anders aufgeführt, sind die Heterocyclen mono- oder polycyclisch, zum Beispiel monocyclisch, bicyclisch oder tricyclisch. Vorzugsweise sind sie monocyclisch oder bicyclisch. Vorzugsweise sind es 5-gliedrige, 6-gliedrige oder 7-gliedrige Ringe, besonders bevorzugt 5-gliedriger oder 6-gliedrige Ringe. Im Fall von polycyclischen Heterocyclen mit 2 und mehr Heteroatomen können diese alle im gleichen Ring vorkommen oder auf mehrere Ringe verteilt sein.

Als Heteroaryl werden in der vorliegenden Erfindung Reste bezeichnet, die von monocyclischen oder bicyclischen, aromatischen Heterocyclen abgeleitet sind. Beispiele für Heteroaryl sind: Pyrrolyl, Furanyl (=Furyl), Thiophenyl (=Thienyl), Imidazolyl, Pyrazolyl, 1,2,3-Triazolyl, 1,2,4-Triazolyl, 1,3-Oxazolyl (=Oxazolyl), 1,2-Oxazolyl (=Isoxazolyl), Oxadiazolyl, 1,3-Thiazolyl (=Thiazolyl), 1,2-Thiazolyl (=Isothiazolyl), Tetrazolyl, Pyridinyl (=Pyridyl) Pyridazinyl, Pyrimidinyl, Pyrazinyl, 1,2,3-Triazinyl, 1,2,4-Triazinyl, 1,3,5-Triazinyl, 1,2,4,5-Tetrazinyl, Indazolyl, Indolyl, Benzothiophenyl, Benzofuranyl, Benzothiazolyl, Benzimidazolyl, Chinolinyl, Isochinolinyl, Chinazolinyl, Chinoxalinyl, Phthalazinyl, Thienothiophenyl, 1,8-Naphthyridinyl, andere Naphthyridinyle, Pteridinyl oder Thiazolo[3,2-b][1,2,4]-triazolyl. Sofern es sich nicht um monocyclische Systeme handelt, ist bei jedem der vorgenannten Heteroaryle für den zweiten Ring auch die gesättigte Form (Perhydroform) oder die teilweise ungesättigte Form (beispielsweise die Dihydroform oder Tetrahydroform) oder die maximal ungesättigte (nicht-aromatische) Form mit umfasst, sofern die jeweiligen Formen bekannt und stabil sind. Die Bezeichnung Heteroaryl umfasst somit in der vorliegenden Erfindung beispielsweise auch bicyclische Reste, in denen sowohl beide Ringe aromatisch sind als auch bicyclische Reste, in denen nur ein Ring aromatisch ist. Solche Beispiele für Heteroaryl sind: 3H-Indolinyl, 2(1H)-Chinolinonyl, 4-Oxo-1,4-dihydrochinolinyl, 2H-1-Oxoisochinolyl, 1,2-Dihydrochinolinyl, 3,4-Dihydrochinolinyl, 1,2-Dihydroisochinolinyl, 3,4-Dihydroisochinolinyl, Chromonyl, Chromanyl, 1,3-Benzodioxolyl, Oxindolyl, 1,2,3,4-Tetrahydroisochinolinyl, 1,2,3,4- Tetrahydrochinolinyl, 5,6-Dihydrochinolyl, 5,6-Dihydroisochinolyl, 5,6,7,8-Tetrahydrochinolinyl oder 5,6,7,8-Tetrahydroisochinolyl.

Als Heterocyclyl werden in der vorliegenden Erfindung Reste bezeichnet, die von monocyclischen oder bicyclischen, nicht-aromatischen Heterocyclen abgeleitet sind. Unter nicht-aromatischen Heterocyclen werden nachfolgend insbesondere Heterocycloalkane (vollständig gesättigte Heterocyclen) sowie Heterocycloalkene (teilweise ungesättigte Heterocyclen) verstanden. Im Fall der Heterocycloalkene werden auch Verbindungen mit zwei oder mehreren Doppelbindungen mit umfasst, die gegebenenfalls auch miteinander konjugiert sein können. Beispiele für Heterocyclyl sind: Pyrrolidinyl, Piperidinyl, Piperazinyl, Imidazolidinyl, Pyrazolidinyl, Isothiazolidinyl, Thiazolidinyl, Isoxazolidinyl, Oxazolidinyl, Tetrahydrofuranyl, Tetrahydrothiophenyl, 1,3-Dioxolanyl, 1,4-Dioxinyl, Pyranyl, Thiopyranyl, Tetrahydro-1,2-Oxazinyl, Tetrahydro-1,3-Oxazinyl, Morpholinyl, Thiomorpholinyl, 1,2-Thiazinyl, 1,3-Thiazinyl, 1,4-Thiazinyl, Azepinyl, 1,2-Diazepinyl, 1,3-Diazepinyl, 1,4-Diazepinyl, 1,3-Oxazepinyl, 1,3-Thiazepinyl, Azepanyl, 2-Oxoazepanyl, 1,2,3,4- Tetrahydropyridinyl, 1,2-Dihydropyridinyl, 1,4-Dihydropyridinyl, 1,2,3,6-Tetrahydropyridinyl, 4(3H)-pyrimidonyl, 1,4,5,6-Tetrahydropyrimidinyl, 2-Pyrrolinyl, 3-Pyrrolinyl, 2-Imidazolinyl, 2-Pyrazolinyl, 3,4-Dihydro-2H-pyranyl, Dihydrofuranyl, 7-Oxabicyclo[2.2.1]heptenyl, Dihydrothiophenyl und Dihydrothiopyranyl. Der Grad der Sättigung von heterocyclischen Gruppen ist in der jeweiligen Definition angezeigt.

Substituenten, die von diesen Heterocyclen abgeleitet sind, können über jedes geeignete Kohlenstoffatom verknüpft sowie mit weiteren Substituenten versehen sein. Reste, die von stickstoffhaltigen Heterocyclen abgeleitet sind, können am entsprechenden Stickstoffatom ein Wasserstoffatom oder einen anderen Substituenten aufweisen. Beispiele umschließen Pyrrol, Imidazol, Pyrrolidin, Morpholin, Piperazinreste usw.. Diese stickstoffhaltigen heterocyclischen Reste können auch über das Ring-Stickstoffatom gebunden sein, insbesondere wenn der entsprechende heterocyclische Rest an ein Kohlenstoffatom gebunden ist. Beispielsweise kann ein Thienylrest als 2-Thienyl oder 3-Thienyl vorliegen, ein Piperidinylrest als 1-Piperidinyl (Piperidino), 2-Piperidinyl, 3-Piperidinyl oder 4-Piperidinyl. Geeignete stickstoffhaltige Heterocyclen können auch als N-Oxide oder als quartemäre Salze, die ein Gegenion aufweisen, das von einer physiologisch annehmbaren Säure abgeleitet ist, vorliegen. Beispielsweise können Pyridylreste als Pyridin-N-oxide vorliegen. Geeignete schwefelhaltige Heterocyclen können auch als S-Oxid oder S,S-Dioxid vorliegen.

Als Aryl werden in der vorliegenden Erfindung Reste bezeichnet, die von monocyclischen oder bicyclischen Aromaten abgeleitet sind, die keine Ringheteroatome enthalten. Sofern es sich nicht um monocyclische Systeme handelt, ist bei der Bezeichnung Aryl für den zweiten Ring auch die gesättigte Form (Perhydroform) oder die teilweise ungesättigte Form (beispielsweise die Dihydroform oder Tetrahydroform) mit umfasst, sofern die jeweiligen Formen bekannt und stabil sind. Die Bezeichnung Aryl umfasst in der vorliegenden Erfindung auch beispielsweise bicyclische Reste, in denen sowohl beide Ringe aromatisch sind als auch bicyclische Reste, in denen nur ein Ring aromatisch ist. Beispiele für Aryl sind: Phenyl, Naphthyl, Indanyl, 1,2-Dihydronaphthenyl, 1,4-Dihydronaphthenyl, Indenyl oder 1,2,3,4-Tetrahydronaphthyl.

Arylalkyl (wie Aryl-(C₁-C₆-alkyl)-) bedeutet, dass ein Alkylrest (wie C₁-C₆-Alkyl) wiederum mit einem Arylrest substituiert ist. Heteroarylalkyl (wie Heteroaryl-(C₁-C₆-alkyl)-) bedeutet, dass ein Alkylrest (wie C₁-C₆-Alkyl) wiederum mit einem Heteroarylrest substituiert ist. Heterocyclylalkyl (wie Heterocycyl-(C₁-C₆-alkyl)-) bedeutet, dass ein Alkylrest (wie C₁-C₆-Alkyl) wiederum mit einem Heterocyclylrest substituiert ist. Für die Definitionen und Substitutionsmöglichkeiten von Alkyl, Heteroaryl, Heterocyclyl und Aryl wird auf die vorstehenden Definitionen verwiesen.

Halogen ist Fluor, Chlor, Brom oder Iod, ist bevorzugt Fluor, Chlor, oder Brom, ist besonders bevorzugt Fluor oder Chlor.

Die vorliegende Erfindung schließt alle stereo-isomeren Formen von Verbindungen gemäß der Formel (I) mit ein. Asymmetrische Kohlenstoffatome in Verbindungen gemäß der Formel (I) können unabhängig voneinander S-Konfigurationen oder R-Konfigurationen aufweisen. Die Erfindung schließt alle möglichen Enantiomere und Diastereomere und Mischungen aus zwei oder mehr Stereo-Isomeren, zum Beispiel Mischungen aus Enantiomeren und/oder Diastereomeren, in allen Mengen und Verhältnissen mit ein. Somit können Verbindungen der vorliegenden Erfindung, die als Enantiomere existieren, in Enantiomeren-Reinform, sowohl als rechtsdrehende als auch linksdrehende Antipoden, in Form von Racematen und in Form von Mischungen der zwei Enantiomeren in allen Verhältnissen vorliegen. Im Fall von Cis/Trans-Isomeren schließt die Erfindung sowohl die Cis-Form, als auch die Trans-Form sowie Mischungen von diesen Formen in allen Verhältnissen mit ein. All diese Formen sind Gegenstand der vorliegenden Erfindung. Die Herstellung der einzelnen Stereo-Isomeren kann, falls gewünscht, durch Trennung eines Gemisches mit herkömmlichen Methoden, zum Beispiel durch Chromatographie oder Kristallisation, durch die Verwendung von stereochemisch einheitlichen Ausgangsmaterialien zur Synthese oder durch stereoselektive Synthese erfolgen. Alternativ kann auch einer Derivatisierung vor der Trennung der Stereo-Isomeren durchgeführt werden. Die Trennung eines Gemisches aus Stereo-Isomeren kann mit den Verbindungen der Formel (I) oder mit den entsprechenden Zwischenprodukten während der Synthese durchgeführt werden. Weiterhin umfasst die vorliegende Erfindung auch alle tautomeren Formen von Verbindungen gemäß Formel (I), insbesondere Keto-/Enoltautomerie, d.h. die entsprechenden Verbindungen können entweder in ihrer Keto-Form oder in ihrer Enolform oder in Mischungen davon in allen Verhältnissen vorliegen.

Sofern die Verbindungen gemäß Formel (I) eine oder mehrere saure oder basische Gruppen enthalten, umfasst die vorliegende Erfindung auch die entsprechenden physiologisch oder toxikologisch verträglichen Salze.

Physiologisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein physiologisch verträgliches Anion oder Kation aufweisen. Geeignete physiologisch verträgliche Säureadditionssalze der erfindungsgemäßen Verbindungen sind Salze anorganischer Säuren, wie Salzsäure, Bromwasserstoff-, Phosphor-, Metaphosphor-, Salpeter-, Sulfon- und Schwefelsäure sowie organischer Säuren, wie z.B. Essigsäure, Theophyllinessigsäure, Methylen-bis-b-oxynaphthon-, Benzolsulfon-, Benzoe-, Zitronen-, Ethansulfon-, Salicyl-, Fumar-, Glucon-, Glykol-, Isethion-, Milch-, Lactobion-, Malein-, Äpfel-, Methansulfon-, Bernstein-, p-Toluolsulfon-, Wein- und Trifluoressigsäure. Geeignete pharmazeutisch verträgliche basische Salze sind Ammoniumsalze, Alkalimetallsalze (wie Natrium- und Kaliumsalze) und Erdalkalisalze (wie Magnesium- und Calciumsalze).

Salze mit einem nicht pharmazeutisch verträglichen Anion gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Sofern die Verbindungen der Formel (I) gleichzeitig saure und basische Gruppen in demselben Molekül enthalten, schließt die vorliegende Erfindung - zusätzlich zu den vorher aufgeführten Salzformen - auch innere Salze oder Betaine (Zwitterionen) mit ein. Die entsprechenden Salze der Verbindungen gemäß Formel (I) können durch herkömmliche Methoden, die dem Fachmann bekannt sind, erhalten werden, beispielsweise durch Umsetzung mit einer organischen oder anorganischen Säure oder Base in einem Lösungsmittel oder Dispergiermittel, oder durch Anionen- oder Kationenaustausch mit anderen Salzen.

Die vorliegende Erfindung schließt darüber hinaus alle Solvate von Verbindungen gemäß Formel (I) mit ein, beispielsweise Hydrate oder Addukte mit Alkohol, aktive Metaboliten von Verbindungen gemäß Formel (I) sowie Derivate, die eine physiologisch annehmbare und abspaltbare Gruppe enthalten, beispielsweise Ester oder Amide.

Der hier verwendete Begriff "physiologisch funktionelles Derivat" bezeichnet jedes physiologisch verträgliche Derivat einer erfindungsgemäßen Verbindung der Formel I, z.B. einen Ester, der bei Verabreichung an einen Säuger, wie z.B. den Menschen, in der Lage ist, (direkt oder indirekt) eine Verbindung der Formel I oder einen aktiven Metaboliten hiervon zu bilden.

Zu den physiologisch funktionellen Derivaten zählen auch Prodrugs der erfindungsgemäßen Verbindungen. Solche Prodrugs können in vivo zu einer erfindungsgemäßen Verbindung metabolisiert werden. Diese Prodrugs können selbst wirksam sein oder nicht und sind ebenfalls Gegenstand der vorliegenden Erfindung.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Verbindungen der Formel (I) sind bevorzugt, die die folgenden Bedeutungen aufweisen:
- R1: ist Wasserstoff, Halogen oder -CF₃;
- R2: ist Wasserstoff, -NH₂, -NH(C₁-C₃-Alkyl), -N(C₁-C₃-Alkyl)₂ oder C₁-C₃-Alkoxy und R1 und R2 können nicht gleichzeitig Wasserstoff sein, wobei Alkyl oder Alkoxy unsubstituiert ist;
- R3: ist unsubstituiertes oder zumindest monosubstituiertes C₁-C₁₀-Alkyl, C₂-C₁₀-Alkenyl, Aryl oder Heterocyclyl, wobei die Substituenten ausgewählt sind aus der Gruppe bestehend aus: Aryl, Heteroaryl, Heterocyclyl, Aryl-(C₁-C₆-alkyl)-, Heterocyclyl-(C₁-C₆-alkyl)-, Heteroaryl-(C₁-C₆-alkyl)-, -O-Aryl, Fluor, Chlor, Brom, -CF₃, -OCF₃, -NO₂, -CN, -C(O)R7, Hydroxy, C₁-C₆-Alkyl und C₁-C₃-Alkoxy, und die Aryl-, Heteroaryl- und Heterocyclyl-Fragmente dieser Substituenten können wiederum mit Fluor, Chlor, Brom, Oxo, -CF₃, -OCF₃, -NO_{2,} -CN, Hydroxy, C₁-C₃-Alkyl oder C₁-C₃-Alkoxy zumindest monosubstituiert sein;
- R4 und R5: sind unabhängig voneinander Wasserstoff, unsubstituiertes oder zumindest monosubstituiertes C₁-C₁₀-Alkyl, wobei die Substituenten ausgewählt sind aus: Halogen, C₁-C₆-Alkoxy, -CF₃ oder Hydroxy; oder
R4 und R5 bilden zusammen mit dem Stickstoffatom, an das sie gebunden sind, unsubstituiertes oder zumindest monosubstituiertes Heterocyclyl, wobei die Substituenten ausgewählt sind aus: Halogen, C₁-C₆-Alkyl, C₁-C₆-Alkoxy, -CF₃ oder Hydroxy;
- R6: ist Wasserstoff, -SO₂(C₁-C₃-Alkyl), unsubstituiertes oder zumindest monosubstituiertes C₁-C₁₀-Alkyl, C₂-C₁₀-Alkenyl oder Aryl-(C₁-C₆-alkyl)-, wobei die Substituenten ausgewählt sind aus: Halogen, C₁-C₆-Alkyl, C₁-C₆-Alkoxy, -CF₃ oder Hydroxy; oder R6 bildet zusammen mit R3 und X, sofern X gleich N ist, unsubstituiertes oder zumindest monosubstituiertes Heterocyclyl, wobei die Substituenten ausgewählt sind aus der Gruppe bestehend aus: Aryl, Heteroaryl, Heterocyclyl, Aryl-(C₁-C₆-alkyl)-, Heterocyclyl-(C₁-C₆-alkyl)-, Heteroaryl-(C₁-C₆-alkyl)-, -O-Aryl, Fluor, Chlor, Brom, -CF₃, -OCF₃, -NO₂, -CN, -C(O)R7, Hydroxy, C₁-C₆-Alkyl und C₁-C₃-Alkoxy, und die Aryl-, Heteroaryl- und Heterocyclyl-Fragmente dieser Substituenten können wiederum mit Fluor, Chlor, Brom, Oxo, -CF₃, -OCF₃, -NO₂, -CN, Hydroxy, C₁-C₃-Alkyl oder C₁-C₃-Alkoxy zumindest monosubstituiert sein; R7 ist C₁-C₃-Alkoxy, -O-Phenyl, C₁-C₃-Alkyl, -NH₂, -NH(C₁-C₃-Alkyl), -N(C₁-C₃-Alkyl)₂ oder Phenyl, und die Phenyl-Fragmente von R7 können wiederum mit Fluor, Chlor, Brom, -CF₃, -OCF₃, -NO₂, -CN, Hydroxy, C₁-C₃-Alkyl oder C₁-C₃-Alkoxy zumindest monosubstituiert sein;
- A: ist SO₂, CO oder CH₂;
- X: ist NR6 oder O;
Heteroaryl ist ein 5 bis 10-gliedriger, aromatischer, mono- oder bicyclischer Heterocyclus, der ein oder mehrere Heteroatome ausgewählt aus N, O und S enthält; Heteroaryl ist bevorzugt Pyridinyl, Thienyl, Pyrimidinyl, Imidazolyl, Furanyl, Benzimidazolyl, Pyrazolyl, Thiazolyl, Isoxazolyl, Pyrrolyl, Pyrazinyl, 3-Oxo-1,3-dihydroisobenzofuranyl oder 4,5,6,7-Tetrahydrobenzothiazolyl; Heteroaryl ist besonders bevorzugt Pyridinyl, Thienyl, Pyrazolyl, Furanyl oder Benzimidazolyl;
Aryl ist ein 5 bis 10-gliedriger, aromatischer, Mono- oder Bicyclus; Aryl ist bevorzugt Phenyl, Indanyl oder Naphthyl; Aryl ist besonders bevorzugt Phenyl;
Heterocyclyl ist ein 5 bis 10-gliedriger, nicht-aromatischer, mono- oder bicyclischer Heterocyclus, der ein oder mehrere Heteroatome ausgewählt aus N, O und S enthält; Heterocyclyl ist bevorzugt. Morpholinyl, Pyrrolidinyl, Piperidinyl, Tetrahydropyranyl, Thiazolidinyl, Dihydroisoxazolyl, Piperazinyl oder Tetrahydrofuranyl; Heterocyclyl ist besonders bevorzugt Morpholinyl, Pyrrolidinyl, Piperidinyl oder Piperazinyl;
oder ein physiologisch verträgliches Salz davon;

Mehr bevorzugte Verbindungen der Formel (I) weisen die folgenden Bedeutungen auf:
- R1: ist Wasserstoff, Halogen oder -CF₃;
- R2: ist Wasserstoff, -NH₂, -NH(C₁-C₃-Alkyl), -N(C₁-C₃-Alkyl)₂ oder C₁-C₃-Alkoxy und R1 und R2 können nicht gleichzeitig Wasserstoff sein, wobei Alkyl oder Alkoxy unsubstituiert ist;
- R3: ist unsubstituiertes oder zumindest monosubstituiertes C₁-C₁₀-Alkyl, C₂-C₆-Alkenyl, Aryl oder Heterocyclyl, wobei die Substituenten ausgewählt sind aus der Gruppe bestehend aus: Aryl, Aryl-(C₁-C₆-alkyl)-, Fluor, Chlor, Brom, -CF₃, -C(O)O-(C₁-C₃-Alkyl), - C(O)O-Phenyl, Hydroxy, C₁-C₃-Alkyl und C₁-C₃-Alkoxy, und die Aryl-Fragmente dieser Substituenten können wiederum mit Fluor, Chlor, Brom, Oxo, -CF₃, Hydroxy, C₁-C₃-Alkyl oder C₁-C₃-Alkoxy zumindest monosubstituiert sein;
- R4 und R5: sind unabhängig voneinander Wasserstoff, unsubstituiertes oder zumindest monosubstituiertes C₁-C₁₀-Alkyl, wobei die Substituenten ausgewählt sind aus: Halogen, C₁-C₆-Alkoxy, -CF₃ oder Hydroxy oder R4 und R5 bilden zusammen mit dem Stickstoffatom, an das sie gebunden sind, unsubstituiertes oder zumindest monosubstituiertes Heterocyclyl, wobei die Substituenten ausgewählt sind aus: Halogen, C₁-C₆-Alkyl, C₁-C₆-Alkoxy, -CF₃ oder Hydroxy;
- R6: ist Wasserstoff, -SO₂(C₁-C₃-Alkyl), unsubstituiertes oder zumindest monosubstituiertes C₁-C₁₀-Alkyl oder C₂-C₆-Alkenyl, wobei die Substituenten ausgewählt sind aus: Halogen, C₁-C₆-Alkoxy oder -CF₃; oder R6 bildet zusammen mit R3 und X, sofern X gleich N ist, unsubstituiertes oder zumindest monosubstituiertes Heterocyclyl, wobei die Substituenten ausgewählt sind aus der Gruppe bestehend aus: Aryl, Aryl-(C₁-C₆-alkyl)-, Fluor, Chlor, Brom, -CF₃, -C(O)O-(C₁-C₃-Alkyl), - C(O)O-Phenyl, Hydroxy, C₁-C₃-Alkyl und C₁-C₃-Alkoxy, und die Aryl-Fragmente dieser Substituenten können wiederum mit Fluor, Chlor, Brom, Oxo, -CF₃, Hydroxy, C₁-C₃-Alkyl oder C₁-C₃-Alkoxy zumindest monosubstituiert sein;
- A: ist SO_{2,} CO oder CH₂;
- X: ist NR6 oder O;
Aryl ist Phenyl, Indanyl oder Naphthyl; Aryl ist besonders bevorzugt Phenyl;
Heterocyclyl ist Morpholinyl, Pyrrolidinyl, Piperidinyl, Tetrahydropyranyl, Thiazolidinyl, Dihydroisoxazolyl, Piperazinyl oder Tetrahydrofuranyl; Heterocyclyl ist besonders bevorzugt Morpholinyl, Pyrrolidinyl, Piperidinyl oder Piperazinyl;
oder ein physiologisch verträgliches Salz davon.

Noch mehr bevorzugte Verbindungen der Formel (I) weisen die folgenden Bedeutungen auf:
- R1: ist Chlor, Fluor oder -CF₃;
- R2: ist Wasserstoff, -NH₂ oder C₁-C₃-Alkoxy, wobei Alkoxy unsubstituiert ist;
- R3: ist unsubstituiertes oder zumindest monosubstituiertes C₁-C₁₀-Alkyl, C₂-C₆-Alkenyl, Phenyl oder Heterocyclyl, wobei die Substituenten ausgewählt sind aus der Gruppe bestehend aus: Phenyl, Phenyl-(C₁-C₆-alkyl)-, Fluor, Chlor, Brom, -CF₃, -C(O)O-(C₁-C₃-Alkyl), Hydroxy, C₁-C₃-Alkyl und C₁-C₃-Alkoxy, und die Phenyl-Fragmente dieser Substituenten können wiederum mit Fluor, Chlor, Brom, Oxo, -CF₃, Hydroxy, C₁-C₃-Alkyl oder C₁-C₃-Alkoxy zumindest monosubstituiert sein;
- R4 und R5: sind unabhängig voneinander C₁-C₆-Alkyl; oder R4 und R5 bilden zusammen mit dem Stickstoffatom, an das sie gebunden sind, unsubstituiertes oder zumindest monosubstituiertes Heterocyclyl, wobei die Substituenten ausgewählt sind aus: Halogen, C₁-C₆-Alkyl, C₁-C₆-Alkoxy, -CF₃ oder Hydroxy;
- R6: ist Wasserstoff, -SO₂(C₁-C₃-Alkyl), unsubstituiertes oder zumindest monosubstituiertes C₁-C₆-Alkyl oder C₂-C₃-Alkenyl,
- R6: ist Wasserstoff, -SO₂(C₁-C₃-Alkyl), unsubstituiertes oder zumindest monosubstituiertes C₁-C₆-Alkyl oder C₂-C₃-Alkenyl, wobei die Substituenten ausgewählt sind aus: Halogen, C₁-C₆-Alkoxy oder -CF₃; oder R6 bildet zusammen mit R3 und X, sofern X gleich N ist, unsubstituiertes oder zumindest monosubstituiertes Heterocyclyl, wobei die Substituenten ausgewählt sind aus der Gruppe bestehend aus: Phenyl, Phenyl-(C₁-C₆-alkyl)-, Fluor, Chlor, Brom, -CF₃, -C(O)O-(C₁-C₃-Alkyl), Hydroxy, C₁-C₃-Alkyl und C₁-C₃-Alkoxy, und die Phenyl-Fragmente dieser Substituenten können wiederum mit Fluor, Chlor, Brom, Oxo, -CF₃, Hydroxy, C₁-C₃-Alkyl oder C₁-C₃-Alkoxy zumindest monosubstituiert sein; A ist SO₂, CO oder CH₂;
- X: ist NR6 oder O;
Heterocyclyl ist Morpholinyl, Pyrrolidinyl, Piperidinyl oder Piperazinyl;
oder ein physiologisch verträgliches Salz davon.

Viel mehr bevorzugte Verbindungen der Formel (I) weisen die folgenden Bedeutungen auf:
- R1: ist Chlor oder -CF₃;
- R2: ist Wasserstoff oder -NH₂;
- R3: ist unsubstituiertes oder zumindest monosubstituiertes C₁-C₆-Alkyl, C₂-C₃-Alkenyl oder Phenyl, wobei die Substituenten ausgewählt sind aus: C₁-C₃-Alkyl, C₁-C₃-Alkoxy, Chlor, Fluor oder Hydroxy;
- R4 und R5: sind jeweils Methyl;
- R6: ist Wasserstoff, -SO₂CH₃ oder C₁-C₆-Alkyl;
- A: ist SO₂ oder CH₂;
- X: ist NR6 oder O;
oder ein physiologisch verträgliches Salz davon.

Besonders bevorzugte Verbindungen der Formel (I) sind ausgewählt aus der Gruppe bestehend aus:
6-Chlor-N-dimethylaminomethylen-3-[(3,5-dimethylphenoxy)-methyl]- benzolsulfonamid;
6-Chlor-N-dimethylaminomethylen-3-[(N-(3,3,5-trimethylcyclohexyl)-N-methylsulfonylamino)-methyl]-benzolsulfonamid; 6-Chlor- N-dimethylaminomethylen-3-(3,3,5-trimethylcyclohexylamino-sulfonyl)-benzolsulfonamid; 2-Amino-4-chlor-5-dimethylaminomethylen-sulfamoyl-N-dibutyl-benzolsulfonamid; 2-Amino-4-chlor-5-dimethylaminomethylen-sulfamoyl-N-diisobutyl-benzolsulfonamid; 2-Amino-4-chlor-5-dimethylaminomethylen-sulfamoyl-benzolsulfonsäure-(3,3,5-trimethylcyclohexylamid; 2-Amino-4-chlor-5-dimethylaminomethylen-sulfamoyl-N-cyclohexyl-N-allylbenzolsulfonamid und 2-Amino-4-trifluormethyl-5-dimethylaminomethylen-sulfamoyl-N-cyclohexyl-benzolsulfonamid;

Die Verbindungen der Formel I sind durch unterschiedliche chemische Verfahren herstellbar, die ebenfalls zu der vorliegenden Erfindung gehören. Einige typische Wege sind nachfolgend aufgeführt, wobei sämtliche Substituenten (R, A und X) wie vorstehend angegeben definiert sind, sofern nachfolgend nicht anders aufgeführt ist. Die Ausgangsverbindungen sowie die Zwischenverbindungen (intermediates) sind entweder kommerziell erhältlich oder können mit dem Fachmann bekannten Verfahren hergestellt werden.

Eine gängige Methode zur Herstellung der N-Aminomethylenverbindungen der Formel I besteht in der Umsetzung der Sulfonamide II mit Dialkylformamidacetalen in einem geeigneten Lösungsmittel wie zum Beispiel DMF, Toluol oder Chlorbenzol. Diese Reaktion kann auch analog durchgeführt werden, wenn in der Formel (II) das -A-X-R3-Fragment durch ein -A-Z-Fragment gemäß der nachstehenden Formel (III) ersetzt ist. Das so erhaltene Zwischenprodukt entspricht einer Verbindung gemäß der nachstehenden Formel (III).

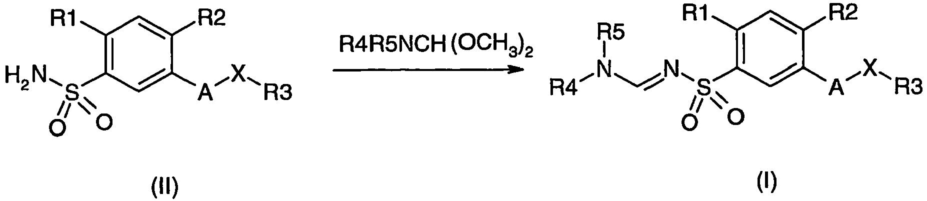

Eine weitere Möglichkeit besteht in der Einführung (Austausch) der R4R5N-Gruppe durch Erhitzen von Verbindungen der allgemeinen Struktur 1, worin R4 und R5 Methyl bedeutet, mit Aminen der allgemeinen Struktur R4R5NH, entweder ohne oder mit einem geeigneten, inerten Lösemittel wie zum Beispiel Toluol, Chlorbenzol, Ethanol, Isopropanol, Butanol.

Der Rest A-X-R3 kann für SO₂-NR3R6 stehen und kann ausgehend von Chlorsufonylverbindungen durch Umsetzung mit Aminen der allgemeinen Formel HNR3R6 erhalten werden. Ganz allgemein lassen sich die Halogenverbindungen der Formel III in die Verbindungen der Formel I durch Umsetzungen mit den Nukleophilen HXR3 umwandeln. Diese Reaktion kann auch analog durchgeführt werden, wenn in der Formel (III) das -SO₂-N=CH-NR4R5-Fragment durch ein -SO₂-NH₂-Fragment ersetzt ist. Das so erhaltene Zwischenprodukt entspricht einer Verbindung gemäß Formel (II).

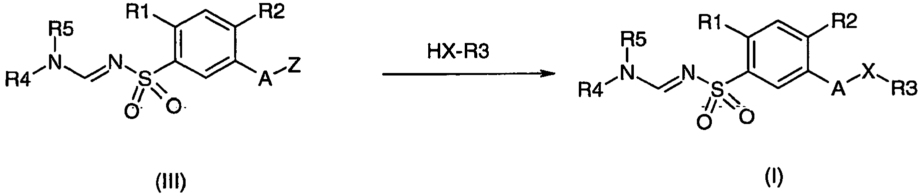

Dabei steht A-Z beispielsweise für COCl, SO₂Cl, SO₂F, CH₂Cl oder CH₂Br und HX-R3 beispielsweise für primäre und sekundäre Amine, Alkohole oder Phenole.

Diese Umsetzungen erfolgen in bekannter Weise in inerten Lösemitteln bei 0-100°C vorzugsweise in Gegenwart von Hilfsbasen, die in der Lage sind, den entstehenden Halogenwasserstoff zu binden.

Die Verbindungen der Formel III können in an sich bekannter Weise durch Halogenierung der entsprechenden Carbonsäuren (-COOH), Alkohole (-CH₂OH) oder Sulfonsäuren (-SO₃H) oder durch Sulfochlorierung mit Chlorsulfonsäure ausgehend von bekannten Vorstufen erhalten werden, wie sie beispielsweise in J. Med. Chem. 29, 1814-20 (1986) oder in DE 2109339 beschrieben sind.

Bei allen Verfahrensweisen kann es angebracht sein, bei bestimmten Reaktionsvorschriften funktionelle Gruppen im Molekül zeitweilig zu schützen. Solche Schutzgruppen sind dem Fachmann geläufig. Die Auswahl einer Schutzgruppe für in Betracht kommende Gruppen und die Verfahren zu ihrer Einführung und Abspaltung sind in der Literatur beschrieben und können gegebenenfalls ohne Schwierigkeiten dem Einzelfall angepasst werden.

Gegenstand der vorliegenden Erfindung ist auch die Verwendung von Verbindungen gemäß der allgemeinen Formel (I) als Arzneimittel beziehungsweise Medikament. Hinsichtlich der Definitionen der Substituenten R1 bis R5 (sowie der weiteren, über die zuvor genannten Substituenten definierten Substituenten), A und X werden auf die Ausführungen hinsichtlich der Verbindung als solche verwiesen.

Die Verwendung von Verbindungen gemäß der allgemeinen Formel (I) als Arzneimittel, wobei die Verbindungen die oben aufgeführten bevorzugten, mehr bevorzugten, noch mehr bevorzugten, viel mehr bevorzugten oder besonders bevorzugten Bedeutungen haben, ist auch ein Gegenstand der vorliegenden Erfindung.

Die erfindungsgemäßen Verbindungen der allgemeinen Formel (I) besitzen eine überraschende hemmende Wirkung an der hormonsensitiven Lipase, HSL, einem allosterischen Enzym in Adipozyten, das durch Insulin gehemmt wird und für den Abbau von Fetten in Fettzellen und damit für die Überführung von Fettbestandteilen in die Blutbahn verantwortlich ist. Eine Hemmung dieses Enzyms entspricht also einer insulinähnlichen Wirkung der erfindungsgemäßen Verbindungen, die letztlich zu einer Verminderung von freien Fettsäuren im Blut und von Blutzucker führt. Sie können also eingesetzt werden bei Entgleisungen des Stoffwechsels wie zum Beispiel bei nicht-insulinabhängiger Diabetes Mellitus (Typ-II-Diabetes Mellitus), beim diabetischen Syndrom und bei Obesitas. Bevorzugt ist die Behandlung von Typ-II-Diabetes Mellitus. Weiterhin eignen sich die erfindungsgemäßen Verbindungen der allgemein Formel (I) auch als Inhibitor der Monoacylglyceridlipase.

In den vorstehenden Ausführungen umfasst der Begriff Behandlung auch die Prophylaxe, Therapie oder Heilung der vorgenannten Krankheiten.

Nachfolgend beziehen sich alle Verweise auf "Verbindung(en) gemäß Formel (I)" auf Verbindung(en) der Formel (I) wie vorstehend beschrieben, sowie ihre Salze, Solvate und physiologisch funktionellen Derivate wie hierin beschrieben.

Die Verbindungen gemäß Formel (I) können Tieren und Menschen, bevorzugt Säugetieren und Menschen, besonders bevorzugt Menschen, verabreicht werden. Die Verbindungen gemäß Formel (I) können dabei selbst als Arzneimittel, in Mischungen miteinander oder in Mischungen mit anderen Arzneimitteln oder in Form von pharmazeutischen Zusammensetzungen verabreicht werden. Folglich sind die Verwendung von Verbindungen gemäß Formel (I) zur Herstellung eines oder mehrerer Medikamente zur Prophylaxe und/oder Behandlung der vorgenannten Krankheiten, pharmazeutische Zusammensetzungen enthaltend eine wirksame Menge von mindestens einer Verbindung gemäß Formel (I) sowie pharmazeutische Zusammensetzungen enthaltend eine wirksame Menge von mindestens einer Verbindung gemäß Formel (I) zur Prophylaxe und/oder Behandlung der vorgenannten Krankheiten ebenfalls Gegenstand der vorliegenden Erfindung

Die Menge einer Verbindung gemäß Formel (I), die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im Allgemeinen liegt die Tagesdosis im Bereich von 0,3 mg bis 100 mg (typischerweise von 3 mg bis 50 mg) pro Tag pro Kilogramm Körpergewicht, z.B. 3-10 mg/kg/Tag. Eine intravenöse Dosis kann z.B. im Bereich von 0,3 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 1,0 bis 1000 mg, typischerweise von 10 bis 600 mg enthalten. Im Falle pharmazeutisch verträglicher Salze beziehen sich die vorgenannten Gewichtsangaben auf das Gewicht der dem Salz zugrunde liegenden freien Verbindung. Zur Prophylaxe oder Therapie der oben genannten Zustände können die Verbindungen gemäß Formel (I) selbst als Verbindung verwendet werden, vorzugsweise liegen sie jedoch mit einem verträglichen Träger in Form einer pharmazeutischen Zusammensetzung vor. Der Träger muss natürlich verträglich sein, in dem Sinne, dass er mit den anderen Bestandteilen der Zusammensetzung kompatibel ist und nicht gesundheitsschädlich für den Patienten ist (physiologisch verträglich). Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel (I). Die erfindungsgemäßen pharmazeutischen Zusammensetzungen können nach einer der bekannten pharmazeutischen Methoden hergestellt werden, die im Wesentlichen darin bestehen, dass die Bestandteile mit pharmakologisch verträglichen Träger- und/oder Hilfsstoffen gemischt werden.

Neben mindestens einer Verbindung gemäß Formel (I) sowie einem oder mehreren Trägerstoffen können die erfindungsgemäßen pharmazeutischen Zusammensetzungen auch Hilfsstoffe enthalten. Beispielsweise eignen sich als Hilfs- beziehungsweise Zusatzstoffe: Füllstoffe, Bindemittel, Gleitmittel, Netzmittel, Stabilisatoren, Emulgatoren, Dispergiermittel, Konservierungsmittel, Süßstoffe, Farbstoffe, Geschmacksstoffe, Aromastoffe, Verdickungsmittel, Verdünnungsmittel, Puffersubstanzen, Lösungsmittel, Lösungsvermittler, Mittel, mit denen eine Depotwirkung erzielt werden kann, Salze zur Veränderung des osmotischen Druckes, Beschichtungsmittel oder Antioxidantien.

Die erfindungsgemäßen pharmazeutischen Zusammensetzungen können beispielsweise in Form einer Pille, Tablette, beschichteten Tablette, Lutschtablette, Granulat, Kapsel, harten oder weichen Gelatinkapsel, wässrigen Lösung, alkoholischen Lösung, ölartigen Lösung, Sirup, Emulsion, Suspension, Zäpfchen, Pastille, Lösung zur Injektion oder Infusion, Salbe, Tinktur, Creme, Lotion, Puder, Spray, transdermalen therapeutischen Systems, Nasenspray, Aerosol, Aerosol-Mischung, Mikrokapsel, Implantat, Stab (rod) oder Pflaster vorliegen.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel (I) abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Polyvinylacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel (I) enthalten; als Pulver (Gelatinekapseln oder Beutel) oder Granulate; als Lösung oder Suspension in einer wässrigen oder nicht-wässrigen Flüssigkeit; oder als eine Öl-in-Wasser- oder Wasserin-Öl-Emulsion. Diese Zusammensetzungen können, wie bereits erwähnt, nach jeder geeigneten pharmazeutischen Methode zubereitet werden, die einen Schritt umfasst, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpresst oder geformt wird, gegebenenfalls mit einem oder mehreren zusätzlichen Bestandteilen. Gepresste Tabletten können durch Tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden. Als Verdünnungsmittel eignen sich beispielsweise Stärke, Cellulose, Saccharose, Laktose oder Kieselgel. Weiterhin können die erfindungsgemäßen pharmazeutischen Zusammensetzungen auch Substanzen enthalten, bei denen es sich nicht um Verdünnungsmittel handelt, beispielsweise eine oder mehrere Gleitmittel, wie Magnesiumstearat oder Talkum, einen Farbstoff, eine Beschichtung (Dragees) oder einen Lack.

Pharmazeutische Zusammensetzungen, die für eine perorale (sublinguale) Verabreichung geeignet sind, umfassen Lutschtabletten, die eine Verbindung gemäß Formel (I) mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wässrige Zubereitungen einer Verbindung gemäß Formel (I), die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im Allgemeinen von 0,1 bis 5 Gew.-% der aktiven Verbindung.

Bei den sterilen Zusammensetzungen zur parenteralen Verabreichung kann es sich vorzugsweise um wässrige oder nicht wässrige Lösungen, um Suspensionen oder Emulsionen handeln. Als Lösungsmittel bzw. Vehikel lassen sich Wasser, Propylenglykol, Polyethylenglykol und Pflanzenöle, insbesondere Olivenöl, organische Ester für die Injektion, beispielsweise Ölsäureethylester, oder andere geeignete organische Lösungsmittel verwenden. Diese Zusammensetzungen können auch Adjuvantien, insbesondere Netzmittel, Mittel zur Einstellung der Isotonizität, Emulgatoren, Dispersionsmittel und Stabilisatoren enthalten. Die Sterilisierung kann auf mehrere Arten erfolgen, beispielsweise durch eine aseptische Filtration, durch Einbringen von Sterilisierungsmitteln in die Zusammensetzung, durch Bestrahlen oder durch Erhitzen. Die Zusammensetzungen können auch in Form sterilen festen Zusammensetzungen hergestellt werden, die bei der Verwendung in sterilem Wasser oder einem anderen sterilen Injektionsmedium gelöst werden.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel (I) mit einem oder mehreren herkömmlichen festen Trägern, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglykole, Alkohole und Kombinationen von zwei oder mehreren dieser Substanzen verwendet werden. Der Wirkstoff ist im Allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wässrigen Lösung, gelöst und/oder dispergiert in einem Haftmittel oder dispergiert in einem Polymer. Eine geeignete Wirkstoff-Konzentration beträgt ca. 1% bis 35%, vorzugsweise ca. 3% bis 15%. Als eine besondere Möglichkeit kann der Wirkstoff, wie beispielsweise in Pharmaceutical Research, 2(6): 318 (1986) beschrieben, durch Elektrotransport oder Iontophorese freigesetzt werden.

### Kombinationen mit anderen Medikamenten

Die erfindungsgemäßen Verbindungen können allein oder in Kombination mit einer oder mehreren weiteren pharmakologisch wirksamen Substanzen verabreicht werden, die beispielsweise günstige Wirkungen auf Stoffwechselstörungen oder damit häufig assoziierte Erkrankungen haben. Solche Medikamente sind zum Beispiel
1. Blutzuckersenkende Medikamente, Antidiabetika,
2. Wirkstoffe zur Behandlung von Dyslipidemien,
3. Antiatherosklerotische Medikamente,
4. Antiadiposita,
5. Antiinflammatorische Wirkstoffe
6. Wirkstoffe zur Behandlung von malignen Tumoren
7. Antithrombotische Wirkstoffe
8. Wirkstoffe zur Behandlung von Bluthochdruck
9. Wirkstoffe zur Behandlung von Herzinsuffizienz sowie
10. Wirkstoffe zur Behandlung und/oder Prävention von Komplikationen, die von Diabetes verursacht werden oder mit Diabetes assoziiert sind.

Sie können mit den erfindungsgemäßen Verbindungen der Formel (I) insbesondere zur synergistischen Wirkungsverbesserung, kombiniert werden. Die Verabreichung der Wirkstoffkombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen.

### Beispielhaft seien genannt:

### Antidiabetika

Geeignete Antidiabetika sind z.B. die in der Roten Liste 2001, Kapitel 12 oder USP Dictionary of USAN and International Drug Names, US Pharmacopeia, Rockville 2003, offenbart. Antidiabetika umfassen alle Insuline und Insulinderivate, wie z.B. Lantus^{®} (siehe www.lantus.com) oder Apidra^{®}, sowie andere schnell wirkende Insuline (siehe US 6,221,633), GLP-1-Rezeptor Modulatoren, wie in WO 01/04146 beschrieben, oder auch wie z.B. diejenigen, die in WO 98/08871 von Novo Nordisk A/S offenbart wurden.

Die oral wirksamen hypoglykämischen Wirkstoffe umfassen vorzugsweise Sulphonylfharnstoffe, Biguanide, Meglitinide, Oxadiazolidindione, Thiazolidindione, Glukosidase-Inhibitoren, Glukagon-Antagonisten, orale GLP-1-Agonisten, DPP-IV Inhibitoren, Kaliumkanalöffner, wie z.B. diejenigen, die in WO 97/26265 und WO 99/03861 offenbart wurden, Insulin-Sensitizer, Inhibitoren von Leberenzymen, die an der Stimulation der Glukoneogenese und/oder Glykogenolyse beteiligt sind, Modulatoren der Glukoseaufnahme, den Fettstoffwechsel verändernde Verbindungen, die zur Veränderung der Lipidzusammensetzung des Blutes führen, Verbindungen, die die Nahrungsmitteleinnahme oder Nahrungsmittelaufnahme verringern, PPAR - und PXR-Modulatoren und Wirkstoffe, die auf den ATP-abhängigen Kaliumkanal der Betazellen wirken.

Bei einer Ausführungsform der Erfindung werden die Verbindungen der Formel (I) in Kombination mit Insulin verabreicht.

Bei einer Ausführungsform der Erfindung werden die Verbindungen der Formel (I) in Kombination mit Substanzen, die Einfluss haben auf die hepatische Glukoseproduktion verabreicht, wie z.B. Glycogen Phosphorylase Inhibitoren (siehe: WO 01/94300, WO 02/096864, WO 03/084923, WO 03/084922, WO 03/104188)

Bei einer Ausführungsform werden die Verbindungen der Formel (I) in Kombination mit einem Sulphonylharnstoff, wie z.B. Tolbutamid, Glibenclamid, Glipizid oder Glimepirid verabreicht.

Bei einer Ausführungsform werden die Verbindungen der Formel (I) in Kombination mit einem Wirkstoff verabreicht, der auf den ATP-abhängigen Kaliumkanal der Betazellen wirkt, wie z.B. Tolbutamid, Glibenclamid, Glipizid, Glimepirid oder Repaglinid.

Bei einer Ausführungsform werden die Verbindungen der Formel (I) in Kombination mit einem Biguanid, wie z.B. Metformin, verabreicht.

Bei wieder einer Ausführungsform werden die Verbindungen der Formel (I) in Kombination mit einem Meglitinid, wie z.B. Repaglinid, verabreicht.

Bei einer Ausführungsform werden die Verbindungen der Formel (I) in Kombination mit einem Thiazolidindion, wie z.B., Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinylmethoxy]phenyl]methyl]-2,4-thiazolidindion, verabreicht.

Bei einer Ausführungsform werden die Verbindungen der Formel (I) in Kombination mit einem DPPIV Inhibitor, wie z.B. in WO98/19998, WO99/61431, WO99/67278, WO99/67279, WO01/72290, WO 02/38541, WO03/040174 beschrieben, insbesondere P 93/01 (1-Cyclopentyl-3-methyl-1-oxo-2-pentanammonium chlorid), P-31/98, LAF237 (1-[2-[3-Hydroxyadamant-1-ylamino)acetyl]pyrrolidin-2-(S)-carbonitril), TS021 ((2S, 4S)-4-Fluoro-1-[[(2-hydroxy-1,1-dimethylethyl)amino]-acetyl]-pyrrolidin-2-carbonitril monobenzenesulfonat)

Bei einer Ausführungsform der Erfindung werden die Verbindungen der Formel (I) in Kombination mit einem PPARgamma Agonist, wie z.B. Rosiglitazon, Pioglitazon, verabreicht.

Bei einer Aufführungsform werden die Verbindungen der Formel (I) in Kombination mit Verbindungen mit inhibitorischer Wirkung auf SGLT-1 und/oder 2, wie z.B. in PCT/EP03106841, PCT/EP03/13454 und PCT/EP03/13455 direkt oder indirekt offenbart, verabreicht.

Bei einer Ausführungsform werden die Verbindungen der Formel (I) in Kombination mit einem α-Glukosidase-Inhibitor, wie z.B. Miglitol oder Acarbose, verabreicht.

Bei einer Ausführungsform werden die Verbindungen der Formel (I) in Kombination mit mehr als einer der vorstehend genannten Verbindungen, z.B. in Kombination mit einem Sulphonylharnstoff und Metformin, einem Sulphonylharnstoff und Acarbose_{,} Repaglinid und Metformin, Insulin und einem Sulphonylharnstoff, Insulin und Metformin, Insulin und Troglitazon, Insulin und Lovastatin, etc. verabreicht.

### Lipidmodulatoren

Bei einer Ausführungsform der Erfindung werden die Verbindungen der Formel (I) in Kombination mit einem HMGCoA-Reduktase Inhibitor wie Lovastatin , Fluvastatin, Pravastatin, Simvastatin, Ivastatin, Itavastatin, Atorvastatin, Rosuvastatin verabreicht.

Bei einer Ausführungsform der Erfindung werden die Verbindungen der Formel (I) in Kombination mit einem Gallensäureresorptionsinhibitor verabreicht (siehe z.B. US 6,245,744, US 6,221,897, US 6,277,831, EP 0683 773, EP 0683 774).

Bei einer Ausführungsform der Erfindung werden die Verbindungen der Formel (I) in Kombination mit einem polymeren Gallensäureadsorber, wie z.B. Cholestyramin, Colesevelam, verabreicht.

Bei einer Ausführungsform der Erfindung werden die Verbindungen der Formel (I) in Kombination mit einem Cholesterinresorptionsinhibitor, wie z.B. in WO 0250027 beschrieben, oder Ezetimibe, Tiqueside, Pamaqueside verabreicht.

Bei einer Ausführungsform der Erfindung werden die Verbindungen der Formel (I) in Kombination mit einem LDL-Rezeptorinduktor (siehe z.B. US 6,342,512) verabreicht.

Bei einer Ausführungsform werden die Verbindungen der Formel (I) in Kombination mit Ballaststoffen, vorzugsweise unlöslichen Ballaststoffen (siehe z.B. Carob/ Caromax^{®} (Zunft H J; et al., Carob pulp preparation for treatment of hypercholesterolemia, ADVANCES IN THERAPY (2001 Sep-Oct), 18(5), 230-6)); Caromax ist ein Carob enthaltendes Produkt der Fa. Nutrinova, Nutrition Specialties & Food Ingredients GmbH, Industriepark Höchst, 65926 Frankfurt / Main) verabreicht. Die Kombination mit Caromax^{®} kann in einer Zubereitung erfolgen, oder durch getrennte Gabe von Verbindungen der Formel I und Caromax^{®}. Caromax^{®} kann dabei auch in Form von Lebensmitteln, wie z.B. in Backwaren oder Müsliriegeln, verabreicht werden.

Bei einer Ausführungsform der Erfindung werden die Verbindungen der Formel (I) in Kombination mit einem PPARalpha Agonist verabreicht.

Bei einer Ausführungsform der Erfindung werden die Verbindungen der Formel (I) in Kombination mit einem Fibrat, wie z.B. Fenofibrat, Gemfibrozil, Clofibrat, Bezafibrat, verabreicht.

Bei einer Ausführungsform der Erfindung werden die Verbindungen der Formel (I) in Kombination mit Nicotinsäure bzw. Niacin verabreicht.

Bei einer Ausführungsform der Erfindung werden die Verbindungen der Formel (I) in Kombination mit einem CETP-Inhibitor, z.B. CP- 529, 414 (Torcetrapib), verabreicht.

Bei einer Ausführungsform der Erfindung werden die Verbindungen der Formel (I) in Kombination mit einem ACAT-Inhibitor verabreicht

Bei einer Ausführungsform der Erfindung werden die Verbindungen der Formel (I) in Kombination mit einem MTP-Inhibitor, wie z.B. Implitapide, verabreicht.

Bei einer Ausführungsform der Erfindung werden die Verbindungen der Formel (I) in Kombination mit einem Antioxidanz verabreicht.

Bei einer Ausführungsform der Erfindung werden die Verbindungen der Formel (I) in Kombination mit einem Lipoprotein-Lipase Inhibitor, verabreicht.

Bei einer Ausführungsform der Erfindung werden die Verbindungen der Formel (I) in Kombination mit einem ATP-Citrat-Lyase Inhibitor verabreicht.

Bei einer Ausführungsform der Erfindung werden die Verbindungen der Formel (I) in Kombination mit einem Squalen Synthetase Inhibitor verabreicht.

Bei einer Ausführungsform der Erfindung werden die Verbindungen der Formel (I) in Kombination mit einem Lipoprotein(a) Antagonist verabreicht.

### Antiobesita

Bei einer Ausführungsform der Erfindung werden die Verbindungen der Formel (I) in Kombination mit einem Lipase Inhibitor, wie z.B. Orlistat, verabreicht.

Bei einer Ausführungsform ist der weitere Wirkstoff Fenfluramin oder Dexfenfluramin.

Bei noch einer Ausführungsform ist der weitere Wirkstoff Sibutramin.

Bei einer weiteren Ausführungsform werden die Verbindungen der Formel (I) in Kombination mit CART-Modulatoren (siehe "Cocaine-amphetamine-regulated transcript influences energy metabolism, anxiety and gastric emptying in mice" Asakawa, A, et al., M.:Hormone and Metabolic Research (2001), 33(9), 554-558), NPY-Antagonisten z.B. Naphthalin-1-sulfonsäure {4-[(4-amino-quinazolin-2-ylamino)-methyl]-cyclohexylmethyl}- amid; hydrochlorid (CGP 71683A)), MC4-Agonisten (z.B. 1-Amino-1,2,3,4-tetrahydro-naphthalin-2-carbonsäure [2-(3a-benzyl-2-methyl-3-oxo- 2,3,3a,4,6,7-hexahydro-pyrazolo[4,3-c]pyridin-5-yl)-1-(4-chloro-phenyl)-2-oxo-ethyl]- amid; (WO 01/91752)) , Orexin-Antagonisten (z.B. 1-(2-Methyl-benzoxazol-6-yl)-3-[1,5]naphthyridin-4-yl-harnstoff; hydrochloride (SB-334867-A)), H3-Agonisten (3-Cyclohexyl-1-(4,4-dimethyl-1,4,6,7-tetrahydro-imidazo[4,5-c]pyridin-5-yl)-propan-1- on Oxalsäuresalz (WO 00/63208)); TNF-Agonisten, CRF-Antagonisten (z.B. [2-Methyl-9-(2,4,6-trimethyl-phenyl)-9H-1,3,9-triaza-fluoren-4-yl]-dipropyl-amin (WO 00/66585)), CRF BP-Antagonisten (z.B. Urocortin), Urocortin-Agonisten, β3-Agonisten (z.B. 1-(4-Chloro-3-methanesulfonylmethyl-phenyl)-2-[2-(2,3-dimethyl-1H-indol-6-yloxy)-ethylamino]-ethanol; hydrochloride (WO 01/83451)), MSH (Melanocyt-stimulierendes Hormon)-Agonisten, CCK-A Agonisten (z.B. {2-[4-(4-Chloro-2,5-dimethoxy-phenyl)-5-(2-cyclohexyl-ethyl)-thiazol-2-ylcarbamoyl]-5,7- dimethyl-indol-1-yl}-acetic acid Trifluoressigsäuresalz (WO 99/15525)); Serotonin-Wiederaufnahme-Inhibitoren (z.B. Dexfenfluramine), gemischte Serotonin- und noradrenerge Verbindungen (z.B. WO 00/71549), 5HT-Agonisten z.B. 1-(3-Ethyl-benzofuran-7-yl)-piperazin Oxalsäuresalz (WO 01/09111), Bombesin-Agonisten, Galanin-Antagonisten, Wachstumshormon (z.B. humanes Wachstumshormon), Wachstumshormon freisetzende Verbindungen (6-Benzyloxy-1-(2-diisopropylamino-ethylcarbamoyl)-3,4-dihydro-1H-isoquinoline-2-carboxylic acid tert-bütyl ester (WO 01/85695)), TRH-Agonisten (siehe z.B. EP 0 462 884) entkoppelnde Protein 2- oder 3-Modulatoren, Leptinagonisten (siehe z.B. Lee, Daniel W.; Leinung, Matthew C.; Rozhavskaya-Arena, Marina; Grasso, Patricia. Leptin agonists as a potential approach to the treatment of obesity. Drugs of the Future (2001), 26(9), 873-881),

DA-Agonisten (Bromocriptin, Doprexin), Lipase/Amylase-Inhibitoren (z.B. WO 00/40569), PPAR-Modulatoren (z.B. WO 00/78312), RXR-Modulatoren oder TR-β-Agonisten verabreicht.
Bei einer Ansführungsform der Erfindung ist der weitere Wirkstoff Leptin.

Bei einer Ausführungsform ist der weitere Wirkstoff Dexamphetamin, Amphetamin, Mazindol oder Phentermin.

Bei einer Ausführungsform werden die Verbindungen der Formel (I) in Kombination mit Medikamenten mit Wirkungen auf das Herz-Kreislauf- und das Blutgefäß-System, verabreicht, wie z.B. ACE-Hemmer (z.B. Ramipril), Medikamente, die auf das Angiotensin-Renin-System wirken, Calcium-Antagonisten, Beta-Blocker etc.

Bei einer Ausführungsform werden die Verbindungen der Formel (I) in Kombination mit anti-entzündlich wirkenden Medikamenten verabreicht.

Bei einer Ausführungsform werden die Verbindungen der Formel (I) in Kombination mit Medikamenten, die zur Krebstherapie und Krebsprävention eingesetzt werden, verabreicht.

Es versteht sich, dass jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird.

### Experimenteller Teil

Die nachfolgenden Beispiele beschreiben die Erfindung näher, ohne sie einzuschränken.

### Beispiele:

### Beispiel 1:

### 4-Methoxy-3-hydroxymethylphenylsulfonsäure-N-dimethylaminomethylen-amid

Die Mischung bestehend aus 1 g 4-Methoxy-3-hydroxymethyl-phenylsulfonsäure-amid, 0,66 g Dimethylformamid-dimethylacetal und 5 ml Dimethylformamid wird eine Stunde bei 80°C gerührt und anschließend im Vakuum eingeengt. Es verbleibt ein allmählich zu einem Feststoff erstarrendes Öl, das ohne weitere Reinigung für die folgende Chlorierung verwendet wird.

### Beispiel 2:

### 4-Methoxy-3-chlormethyl-phenylsulfonsäure-N-dimethylaminomethylen-amid

Das oben beschriebene Produkt wird in 5 ml Toluol mit 0,4 ml Thionylchlorid versetzt und 3 Stunden bei 60°C gerührt. Nach dem Abziehen der flüchtigen Anteile im Vakuum wird der Rückstand in Essigester mit 1N Natronlauge ausgeschüttelt und die organische Phase mit Wasser nachgewaschen, über Natriumsulfat getrocknet und im Vakuum eingeengt.

### Beispiel 3:

### N-Dimethylaminomethylen-4-methoxy-3-[(3,3,5-trimethyl-cyclohexylamino)-methyl]-benzolsulfonamid

Die Mischung von 0,2 g 4-Methoxy-3-chlormethyl-phenylsulfonsäure-N-dimethylaminomethylen-amid, 0,37 ml 3,3,5-Trimethylcyclohexylamin und 5ml Methylenchlorid wird 24 Stunden bei RT gerührt und dann im Vakuum eingeengt.
Der Rückstand wird in Essigester aufgenommen, mit Wasser ausgeschüttelt, die organische Phase über Natriumsulfat getrocknet und eingeengt.
Fp.: Öl

Sofern nicht anders angegeben, werden die nachfolgenden Beispiele analog zu den Beispielen 1 bis 3 hergestellt.

### Beispiel 4:

### N-Dimethylaminomethylen-4-methoxy-3-[(3,5-dimethyl-phenoxy)-methyl]-benzolsulfonamid

Fp: Öl

### Beispiel 5:

### N-Dimethylaminomethylen-4-methoxy-3-[(3,3,5-trimethyl-cyclohexylamino)-methyl]-N-methylsulfonyl-benzolsulfonamid

Fp: Öl

### Beispiel 6:

### N-Dimethylaminomethylen-4-methoxy-3-dibutylaminomethyl- benzolsulfonamid

Fp: Öl

### Beispiel 7:

### N-Dimethylaminomethylen-4-methoxy-3-(4-benzyl-piperazinomethyl)-benzolsulfonamid

Fp: Öl

### Beispiel 8:

### N-Dimethylaminomethylen-4-methoxy-3-[(3,5-dimethylpiperidino)-methyl]- benzolsulfonamid

Fp:: 89°C

### Beispiel 9:

### N-Dimethylaminomethylen-4-methoxy-3-[(2-benzyl-phenoxy)-methyl]-benzolsulfonamid

Diese Verbindung wird analog dem Beispiel 3 ausgehend von 2-Benzylphenol, Natriumhydrid und 4-Methoxy-3-chlormethyl-phenylsulfonsäure-N-dimethylaminomethylen-amid in Tetrahydrofuran als Öl erhalten.

### Beispiel 10:

### 4-Chlor-3-(dimethylaminomethylen-sulfamoyl)-N-(3,3,5-trimethyl-cyclohexyl)-benzamid

Fp:: 180°C

### Beispiel 11:

### 4-Chlor-3-(dimethylaminomethylen-sulfamoyl)-N-(2,2,6,6-tetramethyl-piperidin-4-yl)-benzamid

Fp:: 192°C

### Beispiel 12:

### 4-Chlor-3-(dimethylaminomethylen-sulfamoyl)-N-(3,,5-dimethyl-phenyl)- benzamid

Fp:: 216°C

### Beispiel 13:

### 4-Chlor-3-(dimethylaminomethylen-sulfamoyl)-benzoesäure-(cis-2,6-dimethylmorpholid)

Fp:: Öl

### Beispiel 14:

### 4-Chlor-3-(dimethylaminomethylen-sulfamoyl)-benzoesäure-(3,3-dimethyl-piperidid)

Fp:: Öl

### Beispiel 15:

### 4-Chlor-3-(dimethylaminomethylen-sulfamoyl)-N-(2,2-dimethyl-propyl)- benzamid

Fp:: Harz

### Beispiel 16:

### 4-Chlor-3-(dimethylaminomethylen-sulfamoyl)-N-(2-chlorphenyl-methyl)- benzamid

Fp:: 167°C

### Beispiel 17:

### 4-Chlor-3-(N-methylpiperazinomethylen-sulfamoyl)-N-(3,3,5-dimethyl-cyclohexyl)-benzamid

Dieses Produkt wird durch Erhitzen von 4-Chlor-3-(dimethylaminomethylen-sulfamoyl)-N-(3,3,5-trimethyl-cyclohexyl)-benzamid mit N-Methylpiperazin in N-Methylpyrrolidon auf 100 °C und durch säulenchromatographische Aufarbeitung (Kieselgel, Methylenchlorid:Methanol=95:5) als gelbes Öl erhalten.

### Beispiel 18:

### 6-Chlor-N-dimethylaminomethylen-3-[(3,3-dimethylpiperidino)-methyl]-benzolsulfonamid

Fp:: Öl

### Beispiel 19:

### 6-Chlor-N-dimethylaminomethylen-3-[(3,5-dimethylphenoxy)-methyl]-benzolsulfonamid

Fp:: 137°C

### Beispiel 20:

### 6-Chlor-N-dimethylaminomethylen-3-[(3,3,5-trimethylcyclohexylamino)-methyl]-benzolsulfonamid

Fp:: Öl

### Beispiel 21:

### 6-Chlor-N-dimethylaminomethylen-3-[(N-(3,3,5-trimethylcyclohexyl)-N-methylsulfonyl-amino)-methyl]- benzolsulfonamid

Diese Verbindung wird durch Umsetzung von 6-Chlor-N-dimethylaminomethylen-3-[(3,3,5-trimethylcyclohexylamino)-methyl]-benzolsulfonamid mit Methansulfonylchlorid in Methylenchlorid in Gegenwart von Pyridin als Hilfsbase und durch säulenchromatografische Reinigung (Kieselgel, Methylenchlorid:Methanol=95:5) als Öl erhalten.

### Beispiel 22:

### 6-Chlor-3-(3,3,5-trimethylcyclohexylamino-sulfonyl)- benzolsulfonamid (Zwischenstufe)

Die Mischung bestehend aus 2,9 g 6-Chlor-3-chlorsufonyl-benzolsulfonamid, 1,4 ml Triethylamin, 1,7 ml 3,3,5-Trimethylcyclohexylamin und 30 ml Tetrahydrofuran wird 24 Stunden bei RT gerührt. Nach dem Einengen im Vakuum wird der Rückstand mit Isopropanol verrührt und der Feststoff abgesaugt, mit Wasser gewaschen und im Vakuum bei 40°C getrocknet.

Fp.: 111°C

### Beispiel 23:

### 6-Chlor-N-dimethylaminomethylen-3-(3,3,5-trimethylcyclohexylamino-sulfonyl]-benzolsulfonamid

wird durch Umsetzung von 6-Chlor-3-(3,3,5-trimethylcyclohexylamino-sulfonyl]-benzolsulfonamid mit Dimethylformamid-dimethylacetal in DMF bei 80°C erhalten.

Fp.: 100°C

### Beispiel 24:

### 6-Chlor-N-dimethylaminomethylen-3-(3,3-dimethylpiperidino-sulfonyl]-benzolsulfonamid

wird analog zu Beispiel 22 und 23 erhalten.

Fp.:123°C

### Beispiel 25:

### 4-Chlor-N-butylaminomethylen-3-[3,3,5-trimethylcyclohexylamino-sulfonyl]-benzolsulfonamid

wird durch Umsetzen von 6-Chlor- N-dimethylaminomethylen-3-(3,3,5-trimethylcyclohexylamino-sulfonyl]-benzolsulfonamid mit Butylamin in N-Methylpyrrolidon erhalten.

Fp.: Öl

### Beispiel 26

### 2-Amino-4-trifluormethyl-5-dimethylaminomethylen-sulfamoyl-N-(3,3,5-trimethylcyclohexyl)-benzolsulfonamid

Fp.: > 165 °C (Zers.)

### Beispiel 27:

### 2-Amino-4-chlor-5-(dimethylaminomethylen-sulfamoyl)-benzolsulfonylchlorid (Zwischenstufe)

4 g 6-Chlor- N-dimethylaminomethylen 1,1-dioxo-1,2,3,4-tetrahydro-5,6-benzo[1,2,4]thiadiazine-7-sulfonamid werden zu Chlorsulfonsäure (15 mL) bei 70° C gegeben und der Ansatz 1.5 h bei 80° C gerührt, anschließend auf Eiswasser getropft und 15 min nachgerührt. Der Feststoff wird abfiltriert und getrocknet und ohne weitere Reinigung für weitere Umsetzungen verwendet.

### Beispiel 28:

### 2-Amino-4-chlor-5-dimethylaminomethylen-sulfamoyl-N-dibutyl-benzolsulfonamid

Eine Lösung von 2-Amino-4-chlor-5-(dimethylaminomethylen-sulfamoyl)-benzolsulfonylchlorid. (120 mg, 0.33 mmol) in Aceton (1 mL) wird mit Dibutylamin versetzt und 1.5 h bei RT gerührt. Der Ansatz wird im Vakuum eingeengt, mit n-Heptan verrieben und abgesaugt.

Fp.: 145 °C(Zers)

### Beispiel 29:

### 2-Amino-4-chlor-5-dimethylanünomethylen-sulfamoyl-N-diisobutylbenzolsulfonamid

Fp.: 126°C

### Beispiel 30:

### 2-Amino-4-chlor-5-dimethylaminomethylen-sulfamoyl-benzolsulfonsäurepiperidid

Fp.: 103°C

### Beispiel 31:

### 2-Amino-4-chlor-5-dimethylaminomethylen₋sulfamoyl-benzolsulfonsäurepiperazid.

Fp.: 96°C

### Beispiel 32:

### 2-Amino-4-chlor-5-dimethylaminomethylen-sulfamoyl-benzolsulfonsäure-(3,3,5-trimethylcyclohexyl)-amid

Fp.: 100°C

### Beispiel 33:

### 2-Amino-4-chlor-5-dimethylaminomethylen-sulfamoyl-N-cyclohexyl-N-allylbenzolsulfonamid

Fp.: 90°C

### Beispiel 34:

### 2-Amino-4-chlor-5-dimethylaminomethylen-sulfamoyl- N-cyclohexyl-N-isopropylbenzolsulfonamid

Fp.: 122°C (Zers)

### Beispiel 35:

### 2-Amino-4-chlor-5-dimethylaminomethylen-sulfamoyl- N-cyclohexyl-N-ethylbenzolsulfonamid

Fp.: Öl

### Beispiel 36:

### 2-Amino-4-chlor-5-dimethylaminomethylen-sulfamoyl- N-(2-methylheptyl)-benzolsulfonamid

Fp.: Öl

### Beispiel 37:

### 2-Amino-4-chlor-5-dimethylaminomethylen-sulfamoyl- N-hexyl-benzolsulfonamid

Fp.: Öl

### Beispiel 38:

### 2-Amino-4-chlor-5-dimethylaminomethylen-sulfamoyl- N-octyl-benzolsulfonamid

Fp.: 110°C

### Beispiel 39:

### 2-Amino-4-chlor-5-dimethylaminomethylen-sulfamoyl- N-nonyl-benzolsulfonamid

Fp.: Öl

### Beispiel 40:

### 2-Amino-4-chlor-5-dimethylaminomethylen-sulfamoyl- N-decyl-benzolsulfonamid

Fp.: Öl

### Beispiel 41:

### 2-Amino-4-trifluormethyl-5-dimethylaminomethylen-sulfamoyl- N-decyl-benzolsulfonamid

Fp.: Öl

### Beispiel 42:

### 2-Amino-4-trifluormethyl-5-dimethylaminomethylen-sulfamoyl- N-nonyl-benzolsulfonamid

Fp.: Öl

### Beispiel 43:

### 2-Amino-4-trifluormethyl-5-dimethylaminomethylen-sulfamoyl- N-octyl-benzolsulfonamid

Fp.: Öl

### Beispiel 44:

### 2-Amino-4-trifluormethyl-5-dimethylaminomethylen-sulfamoyl- N-hexylbenzolsulfonamid

Fp.: Öl

### Beispiel 45:

### 2-Amino-5-dimethylaminomethylen-sulfamoyl- N-cyclohexyl-benzolsulfonamid

Fp.: 156°C

### Beispiel 46:

### 2-Amino-4-chlor-5-dimethylaminomethylen-sulfamoyl- N-cyclopentylbenzolsulfonamid

Fp.: Öl

### Beispiel 47:

### 2-Amino-4-chlor-5-dimethylaminomethylen-sulfamoyl- N-(ethoxicarbonylmethyl)-benzolsulfonamid

Fp.: Öl

### Beispiel 48:

### 2-Amino-4-trifluormethyl-5-dimethylaminomethylen-sulfamoyl- N-cyclohexylbenzolsulfonamid

Fp.: >185°C (Zers.)

### Biochemische Untersuchungen

Die Wirkung der erfindungsgemäßen Verbindungen der Formel I wird an folgenden Enzymtestsystemen dargelegt.

### Enzympräparation:

### Präparation der partiell gereinigten HSL:

Isolierte Rattenfettzellen werden aus Nebenhodenfettgewebe von nicht-behandelten männlichen Ratten (Wistar, 220-250 g) durch Kollagenasebehandlung gemäß publizierter Verfahren gewonnen (z.B. S. Nilsson et al., Anal. Biochem. 158, 1986, 399 - 407; G. Fredrikson et al., J. Biol. Chem. 256, 1981, 6311- 6320; H. Tornquist et al., J. Biol. Chem. 251, 1976, 813 - 819). Die Fettzellen aus 10 Ratten werden dreimal durch Flotation mit jeweils 50 ml Homogenisationspuffer (25 ml Tris/HCl, pH 7.4, 0.25 M Sucrose, 1 mM EDTA, 1 mM DTT, 10 µg/ml Leupeptin, 10 µg/ml Antipain, 20 µg/ml Pepstatin) gewaschen und schließlich in 10 ml Homogenisationspuffer aufgenommen. Die Fettzellen werden im Teflon-in-Glas Homogenisator (Braun-Melsungen) durch 10 Hübe bei 1500 rpm und 15°C homogenisiert. Das Homogenisat wird zentrifugiert (Sorvall SM24-Röhrchen, 5000 rpm, 10 min, 4°C). Der Unterstand zwischen der oben liegenden Fettschicht und dem Pellet wird abgenommen und die Zentrifugation wiederholt. Der daraus resultierende Unterstand wird erneut zentrifugiert (Sorvall SM24-Röhrchen, 20000 rpm, 45 min, 4°C). Der Unterstand wird abgenommen und mit 1 g Heparin-Sepharose (Pharmacia-Biotech, CL-6B, 5 x gewaschen mit 25 mM Tris/HCl, pH 7.4, 150 mM NaCl) versetzt. Nach Inkubation für 60 min bei 4°C (in Intervallen von 15 min aufschütteln) wird der Ansatz zentrifugiert (Sorvall SM24-Röhrchen, 3000 rpm, 10 min, 4°C). Der Überstand wird durch Zugabe von Eisessig auf pH 5.2 gebracht und 30 min bei 4°C inkubiert. Die Präzipitate werden durch Zentrifugation (Sorvall SS34, 12000 rpm, 10 min, 4°C) gesammelt und in 2.5 ml 20 mM Tris/HCl, pH 7.0, 1 mM EDTA, 65 mM NaCl, 13 % sucrose, 1 mM DTT, 10 µg/ml Leupepdn/Pepstatin/Antipain suspendiert. Die Suspension wird über Nacht bei 4°C gegen 25 mM Tris/HCl, pH 7.4, 50 % Glyzerin, 1 mM DTT, 10 µg/ml Leupeptin, Pepstatin, Antipain dialysiert und dann auf eine Hydroxiapatit-Säule aufgetragen (0.1 g pro 1 ml Suspension, äquilibriert mit 10 mM Kaliumphosphat, pH 7.0, 30 % Glyzerin, 1 mM DTT). Die Säule wird mit vier Volumina Äquilibrierungspuffer bei einer Flußrate von 20 bis 30 ml/h gewaschen. Die HSL wird mit einem Volumen Äquilibrierungspuffer, der 0.5 M Kaliumphosphat enthält, eluiert, sodann dialysiert (s.o.) und 5- bis 10-fach konzentriert durch Ultrafiltration (Amicon Diaflo PM 10 Filter) bei 4°C. Die partiell gereinigte HSL kann 4 bis 6 Wochen bei -70°C aufbewahrt werden.

### Substratpräparation:

### Präparation des NAG (NBD-Monoacylglycerid) Substrats:

6 mg Phosphatidylcholin und 6 mg Phosphatidylinositol werden in je 1 ml Chloroform gelöst. 10 mg NAG werden in 1 ml Chlorofom gelöst. Zwei Teile Phosphatidylinositollösung (z.B. 83.5 (µl) und ein Teil Phosphatidylcholinlösung (z.B. 41.5 µl) und 100 µl NAG Lösung werden in Plastikszintillationsgefäßen zusammenpipettiert (Endkonzentration im Test: 0.0375 mg Phospholipid/ml; 0.05 mg/ NAG/ml). Das Chloroform (225 µl Gesamtvolumen) wird durch Überblasen mit einem N₂-Strom komplett entfernt. Das getrocknete Substrat kann für bis zu 3 Tagen bei 4°C aufbewahrt werden. Zur Herstellung der Phospholipidvesikel/Micellen mit interkaliertem NAG (am Testtag) wird das getrocknete Substrat in 20 ml Assaypuffer (25 mM Tris/HCl, pH 7.4; 150 mM NaCl) aufgenommen und zwei Ultraschallbehandlungen mit einem Ultraschallstab (Branson Sonifier Type II, Standardmikrospitze) durchgeführt: 1. Behandlung Einstellung 2, 2 x 1 min, dazwischen jeweils 1 min auf Eis; 2. Behandlung Einstellung 4, 2 x 1 min, dazwischen jeweils 1 min auf Eis. Während dieser Prozedur verändert sich sich die Farbe der Substratlösung von gelb (Extinktionsmaximum 481 nm) nach rot (Extinktionsmaximum 550 nm) durch Interkalation von NAG zwischen die Phospholipidmoleküle der Vesikel/Micellen. Vor Benutzung als Substrat (innerhalb der nächsten 2 h) wird die Lösung noch 15 min auf Eis inkubiert.

### Indirekter NAG Assay:

Der Assay wird in 1.5-ml Eppendorfgefäßen oder 96-Lochplatten für 60 min bei 30°C durchgeführt. Für das Auffinden von Inhibitoren der HSL werden 10 µl der Testsubstanz in Assaypuffer (25 mM Tris/HCl, pH 7.4; 150 mM NaCl) in Gegenwart von 16.6 % DMSO vorgelegt. 180 µl der Substratlösung (20 µg/ml Phosphatidylcholin, 10 µg/ml Phosphatidylinositol, 50 µg/ml NAG in Assaypuffer) werden hinzugegeben. Nach einer Vorinkubation für 15 min bei 30°C werden 20 µl der Enzymlösung in Assaypuffer (1- bis 4-fach verdünnt) hinzupipettiert und die Extinktion bei 480 nm in einem Küvettenphotometer (0.5 ml-Küvette) bzw. Mikrotiterplattenlesegerät sofort gemessen. Nach 60 min Inkubation bei 30°C wird die Extinktion erneut gemessen. Die Zunahme der Extinktion bei 480 nm ist ein Maß für die Enzymaktivität. Unter Standardbedingungen führen 20 µg partiell gereinigte HSL zu einer Extinktionsänderung von 0.4 = 4000 arb. units.

### Direkter NAG Assay:

Alternativ zur Messung der Extinktionsänderung der Substratlösung werden die Produkte der HSL Reaktion durch Phasentrennung/Dünnschichtchromatographie untersucht. Dazu wird der Inkubationsansatz (200 µl Gesamtvolumen, s. indirekter NAG Assay) in 2 ml Eppendorfgefäßen mit 1.3 ml Methanol/Chloroform/Heptan (10:9:7) und anschließend mit 0.4 ml 0.1 M NaOH versetzt. Nach intensivem Mischen (10 sec) wird die Phasentrennung durch Zentrifugation (800xg, 20 min, Raumtemperatur) eingeleitet. Von der wässrigen oberen Phase werden äquivalente Volumen (z. B. 0.4 ml) abgenommen und die Extinktion photometrisch bei 481 nm bestimmt. Zur Dünnschichtchromatographie wird die wässrige Phase getrocknet (SpeedVac) und dann in 50 µl Tetrahydrofuran aufgenommen. 5-µl Proben werden auf Silicagel Si-60 Platten (Merck) aufgetragen. Die Chromatographie wird mit 78 ml Diethylether/22 ml Petroläther/1 ml Eisessig als Laufmittel durchgeführt. Die Menge an freigesetzter fluoreszierende NBD-Fettsäure wird durch Phosphorimaging (Molecular Dynamics, Storm 840 und ImageQuant Software) bei einer Anregungswellenlänge von 460 nm und Emissionswellenlänge von 540-560 nm bestimmt.

### Auswertung:

Substanzen werden üblicherweise in vier unabhängigen Ansätzen geprüft. Die Hemmung der enzymatischen Aktivität der HSL durch eine Testsubstanz wird durch den Vergleich mit einer nicht-gehemmten Kontrollreaktion bestimmt. Die Berechnung des IC₅₀-Wertes erfolgt über eine Hemmkurve mit mind. 10 Konzentrationen der Testsubstanz. Für die Analyse der Daten wird das Softwarepaket GRAPHIT, Elsevier-BIOSOFT , benutzt.

In diesem Test zeigten die Verbindungen die folgende Wirkung:

| Verbindung Beispiel Nr. | IC₅₀ (µM) |
|---|---|
| 19 | 0,40 |
| 21 | 0,50 |
| 23 | 0,15 |
| 28 | 0,03 |
| 29 | 0,70 |
| 32 | 0,001 |
| 33 | 0,20 |
| 48 | 0,70 |

## Patentansprüche

1. Verbindung der allgemeinen Formel (I) 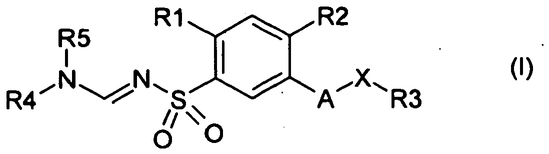 worin bedeuten:
R1 ist Wasserstoff, Halogen oder -CF₃;
R2 ist Wasserstoff, -NH₂, -NH(C₁-C₃-Alkyl), -N(C₁-C₃-Alkyl)₂ oder C₁-C₃-Alkoxy und R1 und R2 können nicht gleichzeitig Wasserstoff sein, wobei Alkyl oder Alkoxy unsubstituiert ist;
R3 ist unsubstituiertes oder zumindest monosubstituiertes C₁-C₁₀-Alkyl, C₂-C₁₀-Alkenyl, Aryl, Heterocyclyl oder Heteroaryl, wobei die Substituenten ausgewählt sind aus der Gruppe bestehend aus: Aryl, Heteroaryl, Heterocyclyl, Aryl-(C₁-C₆-alkyl)-, Heterocyclyl-(C₁-C₆-alkyl)-, Heteroaryl-(C₁-C₆-alkyl)-, -O-Aryl, Fluor, Chlor, Brom, -CF₃, - OCF₃, -NO₂, -CN, -C(O)R7, Hydroxy, C₁-C₆-Alkyl und C₁-C₃-Alkoxy, und die Aryl-, Heteroaryl- und Heterocyclyl-Fragmente dieser Substituenten können wiederum mit Fluor, Chlor, Brom, Oxo, -CF₃, -OCF₃, -NO₂, -CN, Hydroxy, C₁-C₃-Alkyl oder C₁-C₃-Alkoxy zumindest monosubstituiert sein;
R4 und R5 sind unabhängig voneinander Wasserstoff, unsubstituiertes oder zumindest monosubstituiertes C₁-C₁₀-Alkyl oder Aryl-(C₁-C₆-alkyl)-,
wobei die Substituenten ausgewählt sind aus der Gruppe bestehend aus: Aryl, Heteroaryl, Heterocyclyl, Aryl-(C₁-C₆-alkyl)-, Heterocyclyl-(C₁-C₆-alkyl)-, Heteroaryl-(C₁-C₆-alkyl)-, -O-Aryl, Fluor, Chlor, Brom, -CF₃, - OCF₃, -NO₂, -CN, -C(O)R7, Hydroxy, C₁-C₆-Alkyl und C₁-C₃-Alkoxy, und die Aryl-, Heteroaryl- und Heterocyclyl-Fragmente dieser Substituenten können wiederum mit Fluor, Chlor, Brom, Oxo, -CF₃, -OCF₃, -NO₂, -CN, Hydroxy, C₁-C₃-Alkyl oder C₁-C₃-Alkoxy zumindest monosubstituiert sein; oder
R4 und R5 bilden zusammen mit dem Stickstoffatom, an das sie gebunden sind, unsubstituiertes oder zumindest monosubstituiertes Heterocyclyl, wobei die Substituenten ausgewählt sind aus: Halogen, C₁-C₆-Alkyl, C₁-C₆-Alkoxy, Phenyl, Oxo, -NH₂, -NH(C₁-C₃-Alkyl), - N(C₁-C₃-Alkyl)₂, -OCF₃, -CF₃ oder Hydroxy;
R6 ist Wasserstoff, -SO₂(C₁-C₃-Alkyl), unsubstituiertes oder zumindest monosubstituiertes C₁-C₁₀-Alkyl, C₂-C₁₀-Alkenyl oder Aryl-(C₁-C₆-alkyl)-, wobei die Substituenten ausgewählt sind aus: Halogen, C₁-C₆-Alkyl, C₁-C₆-Alkoxy, Phenyl, Oxo, -NH₂, -NH(C₁-C₃-Alkyl), - N(C₁-C₃-Alkyl)₂, -OCF₃, -CF₃ oder Hydroxy; oder R6 bildet zusammen mit R3 und X, sofern X gleich N ist, unsubstituiertes oder zumindest monosubstituiertes Heterocyclyl, wobei die Substituenten ausgewählt sind aus der Gruppe bestehend aus: Aryl, Heteroaryl, Heterocyclyl, Aryl-(C₁-C₆-alkyl)-, Heterocyclyl-(C₁-C₆-alkyl)-, Heteroaryl-(C₁-C₆-alkyl)-, -O-Aryl, Fluor, Chlor, Brom, -CF₃, - OCF₃, -NO₂, -CN, -C(O)R7, Hydroxy, C₁-C₆-Alkyl und C₁-C₃-Alkoxy, und die Aryl-, Heteroaryl- und Heterocyclyl-Fragmente dieser Substituenten können wiederum mit Fluor, Chlor, Brom, Oxo, -CF₃, -OCF₃, -NO₂, -CN, Hydroxy, C₁-C₃-Alkyl oder C₁-C₃-Alkoxy zumindest monosubstituiert sein; R7 ist C₁-C₃-Alkoxy, -O-Phenyl, C₁-C₃-Alkyl, -NH₂, -NH(C₁-C₃-Alkyl), -N(C₁-C₃-Alkyl)₂ oder Phenyl, und die Phenyl-Fragmente von R7 können wiederum mit Fluor, Chlor, Brom, - CF₃, -OCF₃, -NO₂, -CN, Hydroxy, C₁-C₃-Alkyl oder C₁-C₃-Alkoxy zumindest monosubstituiert sein;
A ist SO₂, CO oder CH₂;
X ist NR6 oder O;
Heteroaryl ist ein 5 bis 10-gliedriger, aromatischer, mono- oder bicyclischer Heterocyclus, der ein oder mehrere Heteroatome ausgewählt aus N, O und S enthält;
Aryl ist ein 5 bis 10-gliedriger, aromatischer, Mono- oder Bicyclus;
Heterocyclyl ist ein 5 bis 10-gliedriger, nicht-aromatischer, mono- oder bicyclischer Heterocyclus, der ein oder mehrere Heteroatome ausgewählt aus N, O und S enthält;
oder ein physiologisch verträgliches Salz davon;
unter der Voraussetzung, dass wenn R2 Wasserstoff und R1 Halogen sind, -A-X-R3 nicht i) -C(O)-O-CH₂-CH₂-NH₂, ii) -C(O)-NH-CH₂-CH=CH₂, iii) -C(O)-NH-CH₂-CH₂-Z, mit Z gleich Halogen, Hydroxy, tri-Alkylammonium, Mesylat oder Tosylat, iv) -C(O)-O-(C₁-C₆-Alkyl), v) -C(O)-NH-Benzimidazolyl oder vi) -C(O)-NH-(1,2,3,4-Tetrahydroisochinolinyl) sind, wobei in i) und iii) das -CH₂-CH₂-Fragment 1 bis 4 Alkylreste R¹ bis R⁴ mit jeweils 1 bis 4 C-Atomen aufweisen kann, wobei einer der Reste R¹ bis R⁴ auch eine Carboxy-, Hydroxymethyl- oder eine Alkyloxycarbonyl-Gruppe mit höchstens 5 C-Atomen oder einer oder zwei der Reste R¹ bis R⁴ isoPropyl, iso-Butyl, tert.-Butyl, Phenyl oder Cycloalkyl mit 5 bis 6 C-Atomen bedeuten können, und wobei in v) und vi) das Benzimidazolyl- und das (1,2,3,4-Tetrahydroisochinolinyl)-Fragment unsubstituiert oder zumindest monosubstituiert sein können.

2. Verbindung nach Anspruch 1, worin in der allgemeinen Formel (I) bedeuten:
R1 ist Wasserstoff, Halogen oder -CF₃;
R2 ist Wasserstoff, -NH₂, -NH(C₁-C₃-Alkyl), -N(C₁-C₃-Alkyl)₂ oder C₁-C₃-Alkoxy und R1 und R2 können nicht gleichzeitig Wasserstoff sein, wobei Alkyl oder Alkoxy unsubstituiert ist;
R3 ist unsubstituiertes oder zumindest monosubstituiertes C₁-C₁₀-Alkyl, C₂-C₆-Alkenyl, Aryl oder Heterocyclyl, wobei die Substituenten ausgewählt sind aus der Gruppe bestehend aus: Aryl, Aryl-(C₁-C₆-alkyl)-, Fluor, Chlor, Brom, -CF₃, -C(O)O-(C₁-C₃-Alkyl), - C(O)O-Phenyl, Hydroxy, C₁-C₃-Alkyl und C₁-C₃-Alkoxy;
R4 und sind unabhängig voneinander Wasserstoff, unsubstituiertes oder zumindest monosubstituiertes C₁-C₁₀-Alkyl, wobei die Substituenten ausgewählt sind aus: Halogen, C₁-C₆-Alkoxy, -CF₃ oder Hydroxy oder
R5 R4 und R5 bilden zusammen mit dem Stickstoffatom, an das sie gebunden sind, unsubstituiertes oder zumindest monosubstituiertes Heterocyclyl, wobei die Substituenten ausgewählt sind aus: Halogen, C₁-C₆-Alkyl, C₁-C₆-Alkoxy, -CF₃ oder Hydroxy;
R6 ist Wasserstoff, -SO₂(C₁-C₃-Alkyl), unsubstituiertes oder zumindest monosubstituiertes C₁-C₁₀-Alkyl oder C₂-C₆-Alkenyl, wobei die Substituenten ausgewählt sind aus: Halogen, C₁-C₆-Alkoxy oder -CF₃; oder R6 bildet zusammen mit R3 und X, sofern X gleich N ist, unsubstituiertes oder zumindest monosubstituiertes Heterocyclyl, wobei die Substituenten ausgewählt sind aus der Gruppe bestehend aus: Aryl, Aryl-(C₁-C₆-alkyl)-, Fluor, Chlor, Brom, -CF₃, -C(O)O-(C₁-C₃-Alkyl), - C(O)O-Phenyl, Hydroxy, C₁-C₃-Alkyl und C₁-C₃-Alkoxy, und die Aryl-Fragmente dieser Substituenten können wiederum mit Fluor, Chlor, Brom, Oxo, -CF₃, Hydroxy, C₁-C₃-Alkyl oder C₁-C₃-Alkoxy zumindest monosubstituiert sein;
A ist SO₂, CO oder CH₂;
X ist NR6 oder O;
Aryl ist Phenyl, Indanyl oder Naphthyl;
Heterocyclyl ist Morpholinyl, Pyrrolidinyl, Piperidinyl, Tetrahydropyranyl, Thiazolidinyl, Dihydroisoxazolyl, Piperazinyl oder Tetrahydrofuranyl;
oder ein physiologisch verträgliches Salz davon.

3. Verbindung nach Anspruch 1 oder 2, worin in der allgemeinen Formel (I) bedeuten:
R1 ist Chlor oder -CF₃;
R2 ist Wasserstoff oder -NH₂;
R3 ist unsubstituiertes oder zumindest monosubstituiertes C₁-C₆-Alkyl, C₂-C₃-Alkenyl oder Phenyl, wobei die Substituenten ausgewählt sind aus: C₁-C₃-Alkyl, C₁-C₃-Alkoxy, Chlor, Fluor oder Hydroxy;
R4 und R5 sind jeweils Methyl;
R6 ist Wasserstoff, -SO₂CH₃ oder C₁-C₆-Alkyl;
A ist SO₂ oder CH₂;
X ist NR6 oder O;
oder ein physiologisch verträgliches Salz davon.

4. Verbindung nach einem der Ansprüche 1 bis 3, ausgewählt aus der Gruppe bestehend aus:
6-Chlor-N-dimethylaminomethylen-3-[(3,5-dimethylphenoxy)-methyl]-benzolsulfonamid; 6-Chlor-N-dimethylaminomethylen-3-[(N-(3,3,5-trimethylcyclohexyl)-N-methylsulfonyl-amino)-methyl]- benzolsulfonamid; 6-Chlor-N-dimethylaminomethylen-3-(3,3,5-trimethylcyclohexylamino-sulfonyl)- benzolsulfonamid; 2-Amino-4-chlor-5-dimethylaminomethylen-sulfamoyl-N-dibutyl-benzolsulfonamid; 2-Amino-4-chlor-5-dimethylaminomethylen-sulfamoyl-N-diisobutyl-benzolsulfonamid; 2-Amino-4-chlor-5-dimethylaminomethylen-sulfamoyl-benzolsulfonsäure-(3,3,5-trimethylcyclohexylamid; 2-Amino-4-chlor-5-dimethylaminomethylen-sulfamoyl-N-cyclohexyl-N-allylbenzolsulfonamid und 2-Amino-4-trifluormethyl-5-dimethylaminomethylen-sulfamoyl-N-cyclohexyl-benzolsulfonamid;
oder ein physiologisch verträgliches Salz davon.

5. Verbindung nach einem der Ansprüche 1 bis 4 oder ein physiologisch verträgliches Salz davon zur Verwendung als Arzneimittel.

6. Verwendung einer Verbindung nach einem der Ansprüche 1 bis 4 oder einem physiologisch verträglichen Salz davon zur Herstellung eines Medikaments, das ein Inhibitor der Monoacylglyeridlipase und/oder der hormonsensitiven Lipase (HSL) ist.

7. Verwendung einer Verbindung nach einem der Ansprüche 1 bis 4 oder einem physiologisch verträglichen Salz davon zur Herstellung von einem Medikament zur Prophylaxe und/oder Behandlung von nicht-insulinabhängiger Diabetes Mellitus, diabetischem Syndrom oder Obesitas.

8. Pharmazeutische Zusammensetzung umfassend eine wirksame Menge von mindestens einer Verbindung oder einem physiologisch verträglichen Salz gemäß einem der Ansprüche 1 bis 4 und einem physiologisch verträglichen Träger.

9. Pharmazeutische Zusammensetzung gemäß Anspruch 8, wobei die pharmazeutische Zusammensetzung in Form einer Pille, Tablette, beschichteten Tablette, Lutschtablette, Granulat, Kapsel, harten oder weichen Gelatinkapsel, wässrigen Lösungen, alkoholischen Lösung, ölartigen Lösung, Sirup, Emulsion, Suspension. Pastille, Lösung zur Injektion oder Infusion, Salbe, Tinktur, Creme, Lotion, Puder, Spray, transdermalen therapeutischen Systems, Nasenspray, Aerosol, Aerosol-Mischung, Mikrokapsel, Implantat, Stab oder Pflaster vorliegt.

## Claims

1. A compound of the formula (I) 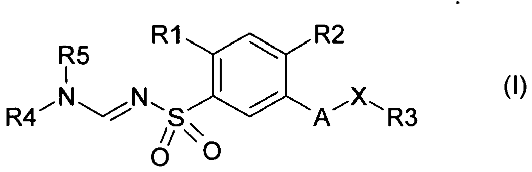 in which the meanings are:
R1 is hydrogen, halogen or -CF₃;
R2 is hydrogen, -NH₂, -NH(C₁-C₃-alkyl), -N(C₁-C₃-alkyl)₂ or C₁-C₃-alkoxy and R1 and R2 may not simultaneously be hydrogen, where alkyl or alkoxy is unsubstituted;
R3 is unsubstituted or at least monosubstituted C₁-C₁₀-alkyl, C₂-C₁₀-alkenyl, aryl, heterocyclyl or heteroaryl, where the substituents are selected from the group consisting of: aryl, heteroaryl, heterocyclyl, aryl-(C₁-C₆-alkyl)-, heterocyclyl-(C₁-C₆-alkyl)-, heteroaryl-(C₁-C₆-alkyl)-, -O-aryl, fluorine, chlorine, bromine, -CF₃, -OCF₃, -NO₂, -CN, -C(O)R7, hydroxy, C₁-C₆-alkyl and C₁-C₃-alkoxy, and the aryl, heteroaryl and heterocyclyl fragments of these substituents may in turn be at least monosubstituted by fluorine, chlorine, bromine, oxo, -CF₃, -OCF₃, -NO₂, -CN, hydroxy, C₁-C₃-alkyl or C₁-C₃-alkoxy;
R4 and R5 are independently of one another hydrogen, unsubstituted or at least monosubstituted C₁-C₁₀-alkyl or aryl-(C₁-C₆-alkyl)-, where the substituents are selected from the group consisting of: aryl, heteroaryl, heterocyclyl, aryl-(C₁-C₆-alkyl)-, heterocyclyl-(C₁-C₆-alkyl)-, heteroaryl-(C₁-C₆-alkyl)-, -O-aryl, fluorine, chlorine, bromine, -CF₃, -OCF₃, -NO₂, -CN, -C(O)R7, hydroxy, C₁-C₆-alkyl and Cₗ-C₃-alkoxy, and the aryl, heteroaryl and heterocyclyl fragments of these substituents may in turn be at least monosubstituted by fluorine, chlorine, bromine, oxo, -CF₃, -OCF₃, -NO₂, -CN, hydroxy, C₁-C₃-alkyl or C₁-C₃-alkoxy; or R4 and R5 form together with the nitrogen atom to which they are bonded unsubstituted or at least monosubstituted heterocyclyl, where the substituents are selected from: halogen, C₁-C₆-alkyl, C₁-C₆-alkoxy, phenyl, oxo, -NH₂, -NH(C₁-C₃-alkyl), -N(C₁-C₃-alkyl)₂, -OCF₃, -CF₃ or hydroxy;
R6 is hydrogen, -SO₂(C₁-C₃-alkyl), unsubstituted or at least monosubstituted C₁-C₁₀-alkyl, C₂-C₁₀-alkenyl or aryl-(C₁-C₆-alkyl)-, where the substituents are selected from: halogen, C₁-C₆-alkyl, C₁-C₆-alkoxy, phenyl, oxo, -NH₂, -NH(C₁-C₃-alkyl), -N(C₁-C₃-alkyl)₂, -OCF₃, -CF₃ or hydroxy; or R6 forms together with R3 and X, if X is N, unsubstituted or at least monosubstituted heterocyclyl, where the substituents are selected from the group consisting of: aryl, heteroaryl, heterocyclyl, aryl-(C₁-C₆-alkyl)-, heterocyclyl-(C₁-C₆-alkyl)-, heteroaryl-(C₁-C₆-alkyl)-, -O-aryl, fluorine, chlorine, bromine, -CF₃, -OCF₃, -NO₂, -CN, -C(O)R7, hydroxy, C₁-C₆-alkyl and C₁-C₃-alkoxy, and the aryl, heteroaryl and heterocyclyl fragments of these substituents may in turn be at least monosubstituted by fluorine, chlorine, bromine, oxo, -CF₃, -OCF₃, -NO₂, -CN, hydroxy, C₁-C₃-alkyl or C₁-C₃-alkoxy;
R7 is C₁-C₃-alkoxy, -O-phenyl, C₁-C₃-alkyl, -NH₂, -NH(C₁-C₃-alkyl), - N(C₁-C₃-alkyl)₂ or phenyl, and the phenyl fragments of R7 may in turn be at least monosubstituted by fluorine, chlorine, bromine, -CF₃, -OCF₃, -NO₂, -CN, hydroxy, C₁-C₃-alkyl or C₁-C₃-alkoxy;
A is SO₂, CO or CH₂;
X is NR6 or O;
heteroaryl is a 5 to 10-membered, aromatic, mono- or bicyclic heterocycle which comprises one or more heteroatoms selected from N, O and S;
aryl is a 5 to 10-membered aromatic mono- or bicyclic system;
heterocyclyl is a 5 to 10-membered, nonaromatic, mono- or bicyclic heterocycle which comprises one or more heteroatoms selected from N, O and S;
or a physiologically tolerated salt thereof;
with the proviso that when R2 is hydrogen and R1 is halogen, then -A-X-R3 is not i) -C(O)-O-CH₂-CH₂-NH₂, ii) -C(O)-NH-CH₂-CH=CH₂, iii) -C(O)-NH-CH₂-CH₂-Z with Z equal to halogen, hydroxy, trialkylammonium, mesylate or tosylate, iv) -C(O)-O-(C₁-C₆-alkyl), v) -C(O)-NH-benzimidazolyl or vi) -C(O)-NH-(1,2,3,4-tetrahydroisoquinolinyl), where the -CH₂-CH₂- fragment in i) and iii) may have 1 to 4 alkyl radicals R¹ to R⁴ each having 1 to 4 carbon atoms, where one of the radicals R¹ to R⁴ may also be a carboxy, hydroxymethyl or an alkyloxycarbonyl group having not more than 5 carbon atoms or one or two of the radicals R¹ to R⁴ may be isopropyl, isobutyl, tert-butyl, phenyl or cycloalkyl having 5 to 6 carbon atoms, and where the benzimidazolyl and the (1,2,3,4-tetrahydroisoquinolinyl) fragment in v) and vi) may be unsubstituted or at least monosubstituted.

2. The compound as claimed in claim 1, where the meanings in the formula (I) are:
R1 is hydrogen, halogen or -CF₃;
R2 is hydrogen, -NH₂, -NH(C₁-C₃-alkyl), -N(C₁-C₃-alkyl)₂ or C₁-C₃-alkoxy and R1 and R2 may not simultaneously be hydrogen, where alkyl or alkoxy is unsubstituted;
R3 is unsubstituted or at least monosubstituted C₁-C₁₀-alkyl, C₂-C₆-alkenyl, aryl or heterocyclyl, where the substituents are selected from the group consisting of: aryl, aryl-(C₁-C₆-alkyl)-, fluorine, chlorine, bromine, -CF₃, -C(O)O-(C₁-C₃-alkyl), -C(O)O-phenyl, hydroxy, C₁-C₃-alkyl and C₁-C₃-alkoxy;
R4 are independently of one another hydrogen, unsubstituted or at least monosubstituted C₁-C₁₀-alkyl,
and R5 where the substituents are selected from: halogen, C₁-C₆-alkoxy, -CF₃ or hydroxy or R4 and R5 form together with the nitrogen atom to which they are bonded unsubstituted or at least monosubstituted heterocyclyl, where the substituents are selected from: halogen, C₁-C₆-alkyl, C₁-C₆-alkoxy, -CF₃ or hydroxy;
R6 is hydrogen, -SO₂(C₁-C₃-alkyl), unsubstituted or at least monosubstituted C₁-C₁₀-alkyl or C₂-C₆-alkenyl, where the substituents are selected from: halogen, C₁-C₆-alkoxy or -CF₃; or R6 forms together with R3 and X, if X is equal to N, unsubstituted or at least monosubstituted heterocyclyl, where the substituents are selected from the group consisting of: aryl, aryl-(C₁-C₆-alkyl)-, fluorine, chlorine, bromine, -CF₃, -C(O)O-(C₁-C₃-alkyl), -C(O)O-phenyl, hydroxy, C₁-C₃-alkyl and C₁-C₃-alkoxy, and the aryl fragments of these substituents may in turn be at least monosubstituted by fluorine, chlorine, bromine, oxo, -CF₃, hydroxy, C₁-C₃-alkyl or C₁-C₃-alkoxy;
A is SO₂, CO or CH₂;
X is NR6 or O;
aryl is phenyl, indanyl or naphthyl;
heterocyclyl is morpholinyl, pyrrolidinyl, piperidinyl, tetrahydropyranyl, thiazolidinyl, dihydroisoxazolyl, piperazinyl or tetrahydrofuranyl;
or a physiologically acceptable salt thereof.

3. The compound as claimed in claim 1 or 2, where the meanings in the formula (I) are:
R1 is chlorine or -CF₃;
R2 is hydrogen or -NH₂;
R3 is unsubstituted or at least monosubstituted C₁-C₆-alkyl, C₂-C₃-alkenyl or phenyl, where the substituents are selected from: C₁-C₃-alkyl, C₁-C₃-alkoxy, chlorine, fluorine or hydroxy;
R4 and R5 are each methyl;
R6 is hydrogen, -SO₂CH₃ or C₁-C₆-alkyl;
A is SO₂ or CH₂;
X is NR6 or O;
or a physiologically acceptable salt thereof.

4. The compound as claimed in any of claims 1 to 3 selected from the group consisting of :
6-chloro-N-dimethylaminomethylene-3-[(3,5-dimethylphenoxy)methyl]benzenesulfonamide; 6-chloro-N-dimethylaminomethylene-3-[(N-(3,3,5-trimethylcyclohexyl)-N-methylsulfonylamino)methyl]benzenesulfonamide; 6-chloro-N-dimethylaminomethylene-3-(3,3,5-trimethylcyclohexylaminosulfonyl)benzenesulfonamide; 2-amino-4-chloro-5-dimethylaminomethylenesulfamoyl-N-dibutylbenzenesulfonamide; 2-amino-4-chloro-5-dimethylaminomethylenesulfamoyl-N-diisobutylbenzenesulfonamide; 2-amino-4-chloro-5-dimethylaminomethylenesulfamoyl-N-(3,3,5-trimethylcyclohexyl)benzenesulfonamide; 2-amino-4-chloro-5-dimethylaminomethylenesulfamoyl-N-cyclohexyl-N-allyl-benzenesulfonamide and 2-amino-4-trifluoromethyl-5-dimethylaminomethylenesulfamoyl-N-cyclohexylbenzenesulfonamide;
or a physiologically acceptable salt thereof.

5. The compound as claimed in any of claims 1 to 4 or a physiologically acceptable salt thereof for use as pharmaceutical.

6. The use of a compound as claimed in any of claims 1 to 4 or a physiologically acceptable salt thereof for producing a medicament which is an inhibitor of monoacylglyeride lipase and/or of hormone-sensitive lipase (HSL).

7. The use of a compound as claimed in any of claims 1 to 4 or a physiologically acceptable salt thereof for producing a medicament for the prophylaxis and/or treatment of non-insulin-dependent diabetes mellitus, diabetic syndrome or obesity.

8. A pharmaceutical composition comprising an effective amount of at least one compound or a physiologically acceptable salt as claimed in any of claims 1 to 4 and a physiologically acceptable carrier.

9. The pharmaceutical composition as claimed in claim 8, where the pharmaceutical composition is in the form of a pill, tablet, coasted tablet, suckable tablet, granules, capsule, hard or soft gelatin capsule, aqueous solutions, alcoholic solution, oily solution, syrup, emulsion, suspension, pastille, solution for injection or infusion, ointment, tincture, cream, lotion, dusting powder, spray, transdermal therapeutic system, nasal spray, aerosol, aerosol mixture, microcapsule, implant, rod or patch.

## Revendications

1. Composé de formule générale (I) 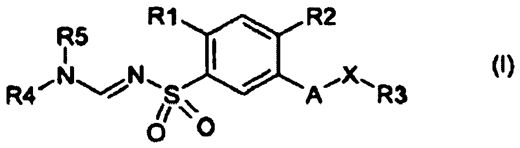 où :
R1 représente hydrogène, halogène ou -CF₃ ;
R2 représente hydrogène, -NH₂, -NH(C₁-C₃-alkyle), -N(C₁-C₃-alkyle)₂ ou C₁-C₃-alcoxy et R1 et R2 ne peuvent pas être simultanément hydrogène ; alkyle ou alcoxy étant non substitués ;
R3 représente C₁-C₁₀-alkyle, C₂-C₁₀-alcényle, aryle, hétérocyclyle ou hétéroaryle non substitués ou au moins monosubstitués, les substituants étant choisis dans le groupe constitué par : aryle, hétéroaryle, hétérocyclyle, aryl-(C₁-C₆-alkyl)-, hétérocyclyl-(C₁-C₆-alkyl)-, hétéroaryl-(C₁-C₆-alkyl)-, -0-aryle, fluor, chlore, brome, -CF₃, -OCF₃, -NO₂, -CN, -C(O)R7, hydroxy, C₁-C₆-alkyle et C₁-C₃-alcoxy, et les fragments aryle, hétéroaryle et hétérocyclyle de ces substituants peuvent être à nouveau au moins monosubstitués par fluor, chlore, brome, oxo, -CF₃, -OCF₃, -NO₂, -CN, hydroxy, C₁-C₃-alkyle ou C₁-C₃-alcoxy ;
R4 et R5 représentent, indépendamment l'un de l'autre hydrogène ; C₁-C₁₀-alkyle ou aryl-(C₁-C₆-alkyl)- non substitués ou au moins monosubstitués, les substituants étant choisis dans le groupe constitué par : aryle, hétéroaryle, hétérocyclyle, aryl-(C₁-C₆-alkyl)-, hétérocyclyl-(C₁-C₆-alkyl)-, hétéroaryl-(C₁-C₆-alkyl)-, -O-aryle, fluor, chlore, brome, -CF₃, -OCF₃, -NO₂, -CN, -C(O)R7, hydroxy, C₁-C₆-alkyle et C₁-C₃-alcoxy, et les fragments aryle, hétéroaryle et hétérocyclyle de ces substituants peuvent être à nouveau au moins monosubstitués par fluor, chlore, brome, oxo, -CF₃, -OCF₃, -NO₂, -CN, hydroxy, C₁-C₃-alkyle ou C₁-C₃-alcoxy ; ou
R4 et R5 forment ensemble avec l'atome d'azote auquel ils sont liés un hétérocyclyle non substitué ou au moins monosubstitué, les substituants étant choisis parmi : halogène, C₁-C₆-alkyle, C₁-C₆-alcoxy, phényle, oxo, -NH₂, -NH(C₁-C₃-alkyle), -N(C₁-C₃-alkyle)₂, -OCF₃, -CF₃ ou hydroxy ;
R6 représente hydrogène, -SO₂(C₁-C₃-alkyle) ; C₁-C₁₀-alkyle, C₂-C₁₀-alcényle ou aryl-(C₁-C₆-alkyl)- non substitués ou au moins monosubstitués, les substituants étant choisis parmi : halogène, C₁-C₆-alkyle, C₁-C₆-alcoxy, phényle, oxo, -NH₂, -NH(C₁-C₃-alkyle), -N(C₁-C₃-alkyle)₂, -OCF₃, -CF₃ ou hydroxy ; ou R6 forme ensemble avec R3 et X, pour autant que X représente N, un hétérocyclyle non substitué ou au moins monosubstitué, les substituants étant choisis dans le groupe constitué par : aryle, hétéroaryle, hétérocyclyle, aryl-(C₁-C₆-alkyl)-, hétérocyclyl-(C₁-C₆-alkyl)-, hétéroaryl-(C₁-C₆-alkyl)-, -O-aryle, fluor, chlore, brome, -CF₃, -OCF₃, -NO₂, -CN, -C(O)R7, hydroxy, C₁-C₆-alkyle et C₁-C₃-alcoxy, et les fragments aryle, hétéroaryle et hétérocyclyle de ces substituants peuvent être à nouveau au moins monosubstitués par fluor, chlore, brome, oxo, -CF₃, -OCF₃, -NO₂, -CN, hydroxy, C₁-C₃-alkyle ou C₁-C₃-alcoxy ;
R7 représente C₁-C₃-alcoxy, -0-phényle, C₁-C₃-alkyle, -NH₂, -NH(C₁-C₃-alkyle), -N(C₁-C₃-alkyle)₂ ou phényle, et les fragments phényle de R7 peuvent être à nouveau au moins monosubstitués par fluor, chlore, brome, -CF₃, -OCF₃, -NO₂, -CN, hydroxy, C₁-C₃-alkyle ou C₁-C₃-alcoxy ;
A représente SO₂, CO ou CH₂ ;
X représente NR6 ou O ;
hétéroaryle représente un hétérocycle monocyclique ou bicyclique aromatique, de 5 à 10 chaînons, qui contient un ou plusieurs hétéroatomes choisis parmi N, O et S ; aryle représente un monocycle ou un bicycle aromatique de 5 à 10 chaînons ;
hétérocyclyle représente un hétérocycle monocyclique ou bicyclique non aromatique, de 5 à 10 chaînons, qui contient un ou plusieurs hétéroatomes choisis parmi N, O et S ; ou un sel physiologiquement acceptable de ceux-ci ;
à condition que lorsque R2 représente hydrogène et R1 représente halogène, -A-X-R3 ne représente pas i)-C(O)-O-CH₂-CH₂-NH₂, ii)-C(O)-NH-CH₂-CH=CH₂, iii)-C(O)-NH-CH₂-CH₂-Z, Z représentant halogène, hydroxy, trialkylammonium, mésylate ou tosylate, iv)-C(O)-O-(C₁-C₆-alkyle), v)-C(O)-NH-benzimidazolyle ou vi)-C(O)-NH-(1,2,3,4-tétrahydro-isoquinoléinyle), où, dans i) et iii) le fragment-CH₂-CH₂- peut présenter 1 à 4 radicaux alkyle R¹ à R⁴ comprenant à chaque fois 1 à 4 atomes de carbone, un des radicaux R¹ à R⁴ pouvant régalement signifier un groupe carboxy, hydroxyméthyle ou alkyloxycarbonyle comprenant au plus 5 atomes de carbone ou un ou deux des radicaux R¹ à R⁴ pouvant signifier iso-propyle, iso-butyle, tert-butyle, phényle ou cycloalkyle comprenant 5 à 6 atomes de carbone, et où, dans v) et vi), le fragment benzimidazolyle et le fragment (1,2,3,4-tétrahydro-isoquinoléinyle) peut être non substitué ou au moins monosubstitué.

2. Composé selon la revendication 1, dans laquelle, dans la formule générale (I) :
R1 représente hydrogène, halogène ou -CF₃ ;
R2 représente hydrogène, -NH₂, -NH(C₁-C₃-alkyle), -N(C₁-C₃-alkyle)₂ ou C₁-C₃-alcoxy et R1 et R2 ne peuvent pas être simultanément hydrogène ; alkyle ou alcoxy étant non substitués ;
R3 représente C₁-C₁₀-alkyle, C₂-C₆-alcényle, aryle ou hétérocyclyle, non substitués ou au moins monosubstitués, les substituants étant choisis dans le groupe constitué par : aryle, aryl-(C₁-C₆-alkyl)-, fluor, chlore, brome, -CF₃, -C (O) 0- (C₁-C₃-alkyle), -C(O)O-phényle, hydroxy, C₁-C₃-alkyle et C₁-C₃-alcoxy ;
R4 et représentent, indépendamment l'un de l'autre hydrogène, C₁-C₁₀-alkyle non substitué ou au moins monosubstitué, les substituants étant choisis dans le groupe constitué par :
R5 halogène, C₁-C₆-alcoxy, -CF₃ ou hydroxy ou R4 et R5 forment ensemble avec l'atome d'azote auquel ils sont liés un hétérocyclyle non substitué ou au moins monosubstitué, les substituants étant choisis parmi : halogène, C₁-C₆-alkyle, C₁-C₆-alcoxy, -CF₃ ou hydroxy ;
R6 représente hydrogène, -SO₂(C₁-C₃-alkyle) ; C₁-C₁₀-alkyle ou C₂-C₆-alcényle non substitués ou au moins monosubstitués, les substituants étant choisis parmi : halogène, C₁-C₆-alcoxy ou -CF₃ ; ou R6 forme ensemble avec R3 et X, pour autant que X représente N, hétérocyclyle non substitué ou au moins monosubstitué, les substituants étant choisis dans le groupe constitué par : aryle, aryl-(C₁-C₆-alkyl)-, fluor, chlore, brome, -CF₃, -C (O) O-(C₁-C₃-alkyle), -C(O)O-phényle, hydroxy, C₁-C₃-alkyle et C₁-C₃-alcoxy, et les fragments aryle de ces substituants peuvent être à nouveau au moins monosubstitués par fluor, chlore, brome, oxo, -CF₃, hydroxy, C₁-C₃-alkyle ou C₁-C₃-alcoxy ;
A représente SO₂, CO ou CH₂ ;
X représente NR6 ou O ;
aryle représente phényle, indanyle ou naphtyle ; hétérocyclyle représente de préférence morpholinyle, pyrrolidinyle, pipéridinyle, tétrahydropyrannyle, thiazolidinyle, dihydro-isoxazolyle, pipérazinyle ou tétrahydrofurannyle ; ou un sel physiologiquement acceptable de celui-ci.

3. Composé selon la revendication 1 ou 2, dans laquelle, dans la formule générale (I) :
R1 représente chlore ou -CF₃ ;
R2 représente hydrogène ou -NH₂ ;
R3 représente C₁-C₆-alkyle, C₂-C₃-alcényle ou phényle non substitués ou au moins monosubstitués, les substituants étant choisis parmi : C₁-C₃-alkyle, C₁-C₃-alcoxy, chlore, fluor ou hydroxy ;
R4 et R5 représentent à chaque fois méthyle ;
R6 représente hydrogène, -SO₂CH₃ ou C₁-C₆-alkyle ;
A représente SO₂ ou CH₂ ;
X représente NR6 ou O ;
ou un sel physiologiquement acceptable de celui-ci.

4. Composé selon l'une quelconque des revendications 1 à 3, choisi dans le groupe constitué par :
le 6-chloro-N-diméthylaminométhylène-3-[(3,5-diméthylphénoxy)-méthyl]-benzènesulfonamide ; le 6-chloro-N-diméthylaminométhylène-3-[(N-(3,3,5-triméthylcyclohexyl)-N-méthylsulfonylamino)-méthyl]-benzènesulfonamide ; le 6-chloro-N-diméthylaminométhylène-3-(3,3,5-triméthylcyclohexylaminosulfonyl)-benzènesulfonamide ;
le 2-amino-4-chloro-5-diméthylaminométhylènesulfamoyl-N-dibutylbenzènesulfonamide ; le 2-amino-4-chloro-5-diméthylaminométhylènesulfamoyl-N-diisobutylbenzènesulfonamide ; le 3,3,5-triméthylcyclohexylamide de l'acide 2-amino-4-chloro-5-diméthylaminométhylènesulfamoylbenzènesulfonique ; le 2-amino-4-chloro-5-diméthylaminométhylènesulfamoyl-N-cyclohexyl-N-allylbenzènesulfonamide et le 2-amino-4-trifluorométhyl-5-diméthylaminométhylènesulfamoyl-N-cyclohexylbenzènesulfonamide ;
ou un sel physiologiquement acceptable de ceux-ci.

5. Composé selon les revendications 1 à 4 ou un sel physiologiquement acceptable de celui-ci destiné à une utilisation comme médicament.

6. Utilisation d'un composé selon l'une quelconque des revendications 1 à 4 ou d'un sel physiologiquement acceptable de celui-ci pour la préparation d'un médicament qui est un inhibiteur de la monoacylglycéride-lipase et/ou de la lipase sensible aux hormones (HSL).

7. Utilisation d'un composé selon l'une quelconque des revendications 1 à 4 ou d'un sel physiologiquement acceptable de celui-ci pour la préparation d'un médicament destiné à la prophylaxie et/ou au traitement du diabète sucré non insulinodépendant, du syndrome diabétique ou de l'obésité.

8. Préparation pharmaceutique, comprenant une quantité active d'au moins un composé ou d'un sel physiologiquement acceptable de celui-ci selon l'une quelconque des revendications 1 à 4 et un support physiologiquement acceptable.

9. Composition pharmaceutique selon la revendication 8, la composition pharmaceutique se trouvant sous forme d'une pilule, d'un comprimé, d'un comprimé revêtu, d'une pastille à sucer, d'un granulat, d'une capsule, d'une capsule de gélatine dure ou molle, de solutions aqueuses, d'une solution alcoolique, d'une solution huileuse, d'un sirop, d'une émulsion, d'une suspension, d'une pastille, d'une solution pour injection ou infusion, d'une pommade, d'une teinture, d'une crème, d'une lotion, d'une poudre, d'un spray, d'un système thérapeutique transdermique, d'un spray nasal, d'un aérosol, d'un mélange pour aérosol, d'une microcapsule, d'un implant, d'une tige ou d'un pansement.
